# EUROPEAN PATENT SPECIFICATION

(11) **EP 4 548 303 B1**
(45) Date of publication and mention of the grant of the patent: **28.10.2026**
(21) Application number: 23911014.1
(22) Date of filing: 30.12.2023
(51) Int. Cl.: G06T 7/80, A61B 6/58, A61B 6/00

(54) **CAMERA CALIBRATION SYSTEMS, METHODS, AND STORAGE MEDIUMS FOR X-RAY IMAGING**
KAMERAKALIBRIERUNGSSYSTEME, VERFAHREN UND SPEICHERMEDIEN ZUR RÖNTGENBILDGEBUNG
SYSTÈMES, PROCÉDÉS ET SUPPORTS DE STOCKAGE D'ÉTALONNAGE DE CAMÉRA POUR L'IMAGERIE PAR RAYONS X

(30) Priority: 30.12.2022 CN 202211738142; 30.12.2022 CN 202223583990 U
(43) Date of publication of application: 07.05.2025
(60) Divisional application: 26206003.1
(73) Proprietor: Shanghai United Imaging Healthcare Co., Ltd., Shanghai 201807 (CN)
(72) Inventor: WANG, Guanqun, Shanghai 201807 (CN); LIU, Wenqiang, Shanghai 201807 (CN); LI, Longhui, Shanghai 201807 (CN)
(74) Representative: Wang, Bo
(86) International application number: PCT/CN2023/143724
(87) International publication number: WO 2024/141102

(56) References cited:
- CN-A- 109 908 497
- CN-A- 112 085 797
- CN-A- 113 040 908
- CN-B- 109 908 497
- CN-U- 219 179 961
- US-A1- 2017 091 940
- US-A1- 2017 219 498
- ALBIOL FRANCISCO ET AL: "Evaluation of modern camera calibration techniques for conventional diagnostic X-ray imaging settings", RADIOLOGICAL PHYSICS AND TECHNOLOGY, SPRINGER JAPAN KK, JP, vol. 10, no. 1, 18 July 2016 (2016-07-18), pages 68 - 81, XP036173613, ISSN: 1865-0333, [retrieved on 20160718], DOI: 10.1007/S12194-016-0369-Y

## Description

### CROSS-REFERENCE TO RELATED APPLICATIONS

This application claims priority to the Chinese Patent Application No. 202211738142.9, filed on December 30, 2022, and the Chinese Patent Application No. 202223583990.5, filed on December 30, 2022.

### TECHNICAL FIELD

The present disclosure relates to a medical field, in particular, relates to camera calibration systems, methods, and storage mediums for X-ray imaging.

### BACKGROUND

During the X-ray imaging, cameras can be applied to an X-ray imaging system for spatial positioning of a target object. In order to achieve a precise spatial positioning of the target object, the camera needs to be calibrated accurately; meanwhile, camera calibration errors may lead to spatial positioning errors in the later stage, thereby affecting the accuracy of X-ray imaging. During the camera calibration, calibration processes and calibration tools may affect the calibration accuracy greatly.

The commonly used camera calibration tools are chessboard, circular array targets, or standard ball array targets of specified sizes, sizes of the calibration targets are usually of standard size and are not suitable for long-distance, large field of view, and other situations, for example, due to an installation distance of the camera being far from the X-ray imaging device, in order for the camera to clearly distinguish a position of the target object, a relatively large calibration target is usually required; moreover, the disassembly and adjustment for the calibration targets are often complex. At the same time, the calibration tools used for a large field of view often face difficulties in processing and fixing, in some scenarios, it is difficult to use large-sized calibration targets. For example, an end of a digital subtraction angiography (DSA) device used for cardiovascular imaging is a "C" arm, which cannot fix large-sized calibration targets. All the problems described above affect an effectiveness of camera calibration, resulting in inaccurate calibration result.

Currently, a conversion relationship (i.e. external parameter) between a robotic arm coordinate system and a camera coordinate system is usually calibrated through a robot arm hand-eye calibration. The manner uses an external camera to identify markers fixed on the calibration targets at the end of the robotic arm to complete the calibration. The external parameter calibration requires two groups of parameters to complete the calculation: one group is image coordinates (i.e. a pixel coordinate) of the marker on the calibration target, and the other group is spatial coordinates of the marker on the calibration target. The accuracy of the two groups of coordinates determines a final camera calibration accuracy. The image coordinates of the marker may be directly collected and obtained based on an image captured by the camera; spatial coordinates values of the marker may be usually obtained by directly measuring a distance between the calibration target and the end of the robotic arm with a ruler, which depends on a relative fixed accuracy and measurement accuracy of the calibration target and the end of the robotic arm, which exists significant errors.

At the same time, factors such as device deformation, installation errors of detectors and tubes can affect the accuracy of X-ray image. The above points make it difficult to fix the calibration target and accurately obtain the spatial coordinates values, as well as how to ensure the accuracy of the final X-ray image.

Therefore, it is desirable to provide a camera calibration method, system, and storage medium for X-ray imaging and a calibration tool to improve accuracy of the camera calibration and ensure the accuracy of X-ray image.

Document US2017/219498A1 discloses a known method of calibration of optical cameras in X-ray imaging systems using calibration markers and multiple acquisition geometries.

### SUMMARY OF THE INVENTION

The invention relates to a camera calibration method, system and computer program in X-ray imaging systems, as defined by the appended independent claims 1, 14 and 15.

### SUMMARY OF THE DISCLOSURE

One aspect of embodiments of the present disclosure may provide a camera calibration method for X-ray imaging implemented on a computing device having one or more processors and one or more storage devices. The method may include: (i) acquiring at least one image taken by a camera to be calibrated, wherein the at least one image includes a calibration target, and the calibration target may includes at least one calibration point; (ii) selecting any calibration point of the at least one calibration point as a target calibration point; (iii) determining, based on the at least one image, image coordinates of the target calibration point; (iv) obtaining a first position and a second position of an X-ray imaging device, wherein when the X-ray imaging device may be located in the first position and the second position, the target calibration point may be within an imaging field of view of the X-ray imaging device, and a first line and a second line may be not parallel, the first line connecting a radiation source and a detector of the X-ray imaging device at the first position, the second line connecting the radiation source and the detector of the X-ray imaging device at the second position; (v) determining, based on the first position and the second position, spatial coordinates of the target calibration point; and (vi) performing calibration on the camera to be calibrated based on the image coordinates and the spatial coordinates.

Another aspect of the present disclosure may provide a calibration target. The calibration target may include at least one calibration unit, each of the at least one calibration unit may include a calibration part and a base plate, and a geometric center of the calibration part may be set as a calibration point, the calibration part may be disposed on the base plate, and there may be an X-ray attenuation difference and an optical imaging difference between the calibration part and the base plate.

Another aspect of the present disclosure may provide a camera calibration device for X-ray imaging. The camera calibration device for X-ray imaging may include the above calibration target.

Another aspect of the present disclosure may provide a system. The system may include at least one storage medium including a set of instructions; at least one processor in communication with the at least one storage medium, wherein when executing the set of instructions, the at least one processor is directed to cause the system to perform operations including: (i) acquiring at least one image taken by a camera to be calibrated, wherein the at least one image includes a calibration target, and the calibration target may include at least one calibration point; (ii) selecting any calibration point of the at least one calibration point as a target calibration point; (iii) determining, based on the at least one image, image coordinates of the target calibration point; (iv) obtaining a first position and a second position of an X-ray imaging device, wherein when the X-ray imaging device may be located in the first position and the second position, the target calibration point may be within an imaging field of view of the X-ray imaging device, and a first line and a second line may be not parallel, the first line connecting a radiation source and a detector of the X-ray imaging device at the first position, the second line connecting the radiation source and the detector of the X-ray imaging device at the second position; (v) determining, based on the first position and the second position, spatial coordinates of the target calibration point; and (vi) performing calibration on the camera to be calibrated based on the image coordinates and the spatial coordinates.

Another aspect of the present disclosure may provide a camera calibration system for X-ray imaging. The camera calibration system for X-ray imaging may include an image acquisition module configured to acquire at least one image taken by a camera to be calibrated, wherein the at least one image may include a calibration target, and the calibration target may include at least one calibration point; a calibration point determination module configured to select any calibration point of the at least one calibration point as a target calibration point; a first coordinate determination module configured to determine, based on the at least one image, image coordinates of the target calibration point; a position acquisition module configured to obtain a first position and a second position of an X-ray imaging device, wherein when the X-ray imaging device may be located in the first position and the second position, the target calibration point may be within an imaging field of view of the X-ray imaging device, and a first line and a second line may be not parallel, the first line connecting a radiation source and a detector of the X-ray imaging device at the first position, the second line connecting the radiation source and the detector of the X-ray imaging device at the second position; a second coordinate determination module configured to determine, based on the first position and the second position, spatial coordinates of the target calibration point; and a camera calibration module configured to perform calibration on the camera to be calibrated based on the image coordinates and the spatial coordinates.

Another aspect of the present disclosure may provide a non-transitory computer readable medium, comprising a set of instructions, wherein when executed by at least one processor, the set of instructions direct the at least one processor to effectuate a method. The method may include (i) acquiring at least one image taken by a camera to be calibrated, wherein the at least one image includes a calibration target, and the calibration target may includes at least one calibration point; (ii) selecting any calibration point of the at least one calibration point as a target calibration point; (iii) determining, based on the at least one image, image coordinates of the target calibration point; (iv) obtaining a first position and a second position of an X-ray imaging device, wherein when the X-ray imaging device may be located in the first position and the second position, the target calibration point may be within an imaging field of view of the X-ray imaging device, and a first line and a second line may be not parallel, the first line connecting a radiation source and a detector of the X-ray imaging device at the first position, the second line connecting the radiation source and the detector of the X-ray imaging device at the second position; (v) determining, based on the first position and the second position, spatial coordinates of the target calibration point; and (vi) performing calibration on the camera to be calibrated based on the image coordinates and the spatial coordinates.

### BRIEF DESCRIPTION OF THE DRAWINGS

This specification will be further illustrated by way of exemplary embodiments, which will be described in detail with the accompanying drawings. These examples are non-limiting, and in these examples, the same number indicates the same structure, wherein:
FIG. 1 is a schematic diagram illustrating an exemplary application scenario of a camera calibration system for X-ray imaging according to some embodiments of the present disclosure;
FIG. 2 is a schematic diagram illustrating exemplary camera calibration system for X-ray imaging according to some embodiments of the present disclosure;
FIG. 3 is a flowchart illustrating an exemplary camera calibration process for X-ray imaging according to some embodiments of the present disclosure;
FIG. 4 is a schematic diagram illustrating an exemplary structure of an X-ray imaging device according to some embodiments of the present disclosure;
FIG. 5A and FIG. 5B are schematic diagrams illustrating an exemplary structure of a relative position between a C-arm and a calibration target according to some embodiments of the present disclosure;
FIG. 6 is a schematic diagram illustrating an exemplary structure of a calibration unit according to some embodiments of the present disclosure;
FIG. 7A and 7B are schematic diagrams illustrating an exemplary calibration target according to some embodiments of the present disclosure;
FIG. 8 is a schematic diagram illustrating an exemplary calibration target under in use according to some embodiments of the present disclosure; and
FIG. 9 is a schematic diagram illustrating an exemplary calibration target under according to some embodiments of the present disclosure.

### DETAILED DESCRIPTION

In order to more clearly illustrate the technical solutions of the embodiments of the present specification, the following briefly introduces the drawings that need to be used in the description of the embodiments. Apparently, the accompanying drawings in the following description are only some examples or embodiments of this specification, and those skilled in the art can also apply this specification to other similar scenarios. Unless obviously obtained from the context or the context illustrates otherwise, the same numeral in the drawings refers to the same structure or operation.

It should be understood that "system," "device," "unit" and/or "module" as used herein is a method for distinguishing different components, elements, parts, parts or assemblies of different levels. However, the words may be replaced by other expressions if other words can achieve the same purpose.

As indicated in the specification and claims, the terms "a," "an," "an" and/or "the" are not specific to the singular and may include the plural unless the context clearly indicates an exception. Generally speaking, the terms "comprising" and "comprising" only suggest the inclusion of clearly identified operations and elements, and these operations and elements do not constitute an exclusive list, and the method or device may also contain other operations or elements.

Flowcharts are used in the present disclosure to illustrate the operation performed by the system according to the embodiment of the present disclosure. It should be understood that the preceding or subsequent operations are not necessarily performed accurately in sequence. Instead, the operations may be processed in reverse order or simultaneously. At the same time, other operations may add to these procedures, or remove one or more operations from these procedures.

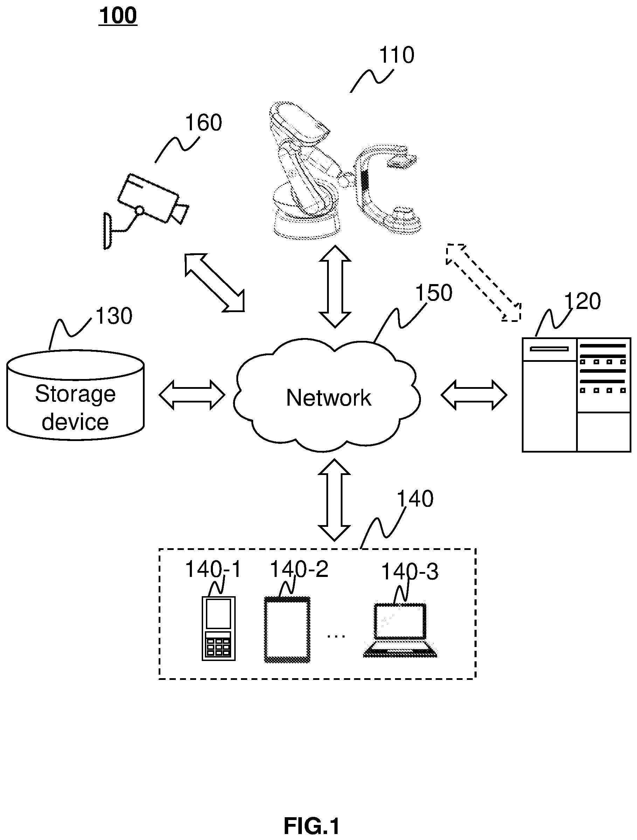

FIG. 1 is a schematic diagram illustrating an exemplary application scenario of a camera calibration system for X-ray imaging according to some embodiments of the present disclosure.

A camera calibration system 100 for X-ray imaging described in the present disclosure refers to as the system 100. As shown in FIG. 1, in some embodiments, the system 100 may include a medical imaging device 110, a processing device 120, a storage device 130, a terminal 140, a network 150, and a camera 160.

The medical imaging device 110 refers to a device in medicine that uses different media to reproduce an internal structure of the human body into an image. In some embodiments, the medical imaging device 110 may include medical devices based on X-ray imaging technology, such as a digital subtraction angiography (DSA) device (e.g. an X-ray imaging device 400), a computed radiography (CR) device, a digital radiography (DR) device, or any combination thereof. The medical imaging device 110 provided above is merely for illustrative purposes, which may not be limited herein. In some embodiments, the medical imaging device 110 may include capturing a target object (e.g., a patient, a phantom, a calibration target, etc.) and sending the image to the processing device 120. The medical imaging device 110 may receive instructions sent by users (e.g., doctors, equipment installation and maintenance personnel, etc.) through the terminal 140, and perform related operations based on the instructions, such as capturing the target object, or the like. In some embodiments, the medical imaging device 110 may exchange data and/or information with other components of the system 100 (e.g., the processing device 120, the storage device 130, the terminal 140) through the network 150. In some embodiments, the medical imaging device 110 may be directly connected with other components of the system 100. In some embodiments, the one or more components of the system 100 (e.g., the processing device 120, the storage device 130) may be included within the medical imaging device 110.

The processing device 120 may process the data and/or information obtained from other components of the device or system and perform the camera calibration process for X-ray imaging based on the data, information, and/or the processing results to complete one or more functions described in some embodiments of the present disclosure. In some embodiments, the processing device 120 may determine two-dimensional coordinates of the target object in the image coordinate system from the image captured by the camera 160, convert the two-dimensional coordinates of the target object into three-dimensional spatial coordinates, determine a spatial position of the target object, and adjust an imaging position of the medical imaging device 110, so that the target object is within an imaging field of view of the medical imaging device 110 or at an isocenter point of the medical imaging device 110.

In some embodiments, the processing device 120 may include one or more sub-processing devices (e.g., single core processing devices or multi-core processing devices). For example, the processing device 120 may include a central processing unit (CPU), a specialized integrated circuit (ASIC), a specialized instruction processor (ASIP), a graphics processor (GPU), a physical processor (PPU), a digital signal processor (DSP), a field programmable gate array (FPGA), a programmable logic circuit (PLD), a controller, a microcontroller unit, a reduced instruction set computer (RISC), a microprocessor, or any combination thereof.

The storage device 130 may be configured to store data or information generated by other devices. In some embodiments, the storage device 130 may store the data and/or information collected by the medical imaging device 110 and/or the camera 160, such as an image captured by the medical imaging device 110 and/or the camera 160. In some embodiments, the storage device 130 may store the data and/or information processed by the processing device 120, such as a spatial position of a camera to be calibrated. The storage device 130 may include one or more storage components, each of which may be an independent device or a part of other devices. The storage device may be local or implemented through the cloud.

The terminal 140 may control operations of the medical imaging device 110 and/or camera 160. The user may issue operation instructions to the medical imaging device 110 and/or camera 160 through the terminal 140 to enable the medical imaging device 110 and/or camera 160 to complete specified operations, such as capturing the target object. In some embodiments, the terminal 140 may instruct the processing device 120 to perform the camera calibration processes for X-ray imaging as shown in some embodiments of the present disclosure. In some embodiments, the terminal 140 may be one or any combination of other devices with input and/or output functions, such as mobile devices 140-1, tablet computers 140-2, laptops 140-3, desktop computers, or the like.

The network 150 may connect various components of the system and/or connect the system with external resource parts. The network 150 may enable communication between various components and with other parts outside the system, promoting an exchange of data and/or information. In some embodiments, the one or more components of the system 100 (e.g., the medical imaging device 110, the processing device 120, the storage device 130, the terminal 140, the camera 160) may send data and/or information to other components through the network 150. In some embodiments, the network 150 may be any one or more of wired or wireless networks.

The camera 160 refers to a device for imaging through optical imaging. In some embodiments, the camera 160 may capture the target object through optical imaging and transmit the image to the processing device 120. In some embodiments, the camera 160 may be installed in a static fixed position to ensure that the target object on the hospital bed can be imaged when the bed is moved to any position. A distance between the camera 160 and the target object is generally greater than a preset value (e.g. 3 meters, 4 meters, etc.). For example, the camera 160 may be installed on a ceiling of a room where the medical imaging device 110 is placed.

It should be noted that the above description is provided for illustrative purposes only and is not intended to limit the scope of the present disclosure. For ordinary technical personnel in this field, various changes and modifications can be made under the guidance of the content of the present disclosure. The features, structures, methods, and other features of the exemplary embodiments described in the present disclosure can be combined in various ways to obtain additional and/or alternative exemplary embodiments. For example, the processing device 120 may be based on cloud computing platforms, such as public clouds, private clouds, communities, and hybrid clouds. However, these changes and modifications will not deviate from the scope of the present disclosure.

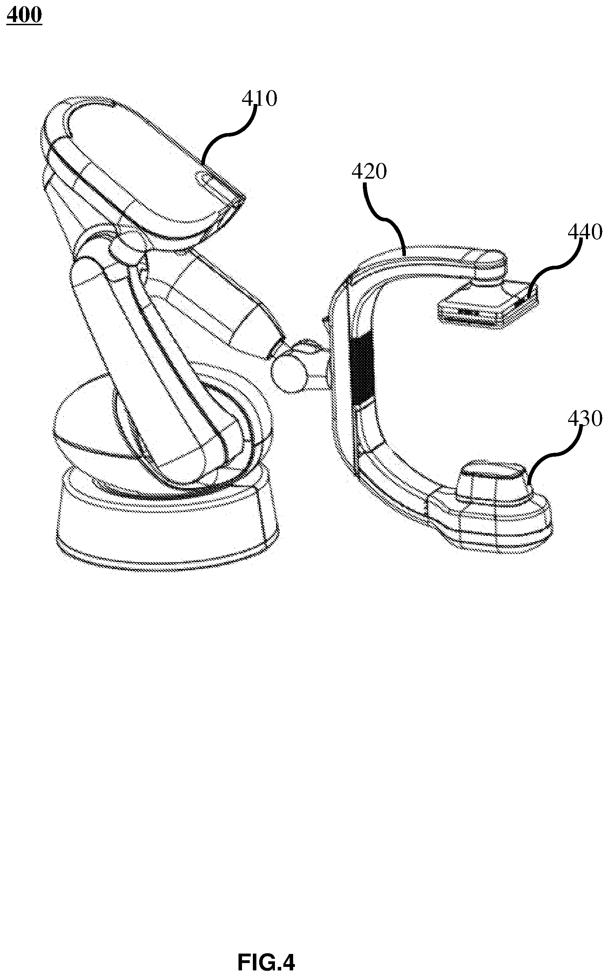

FIG. 4 is a schematic diagram illustrating an exemplary structure of an X-ray imaging device according to some embodiments of the present disclosure. As shown in FIG. 4, an X-ray imaging device 400 may include an X-ray camera, and the X-ray camera may include a radiation source 430 and a detector 440 installed on a C-arm 420. The C-arm 420 may be installed on a robotic arm 410, and the robotic arm 410 may drive the C-arm 420 to move. When taking photos, an X-ray emitted by the radiation source 430 pass through the detected object (i.e. the target object) and reach the detector 440, forming an image on a display screen or X-ray film. A system software of the X-ray imaging device 400 may provide real-time feedback on a real-time coordinate of an intersection point between the detected object and a line connecting the radiation source 430 and the detector 440. Even when the C-arm 420 is moving, the system software of the X-ray imaging device 400 may provide real-time feedback on spatial coordinates (i.e. the three-dimensional coordinates) of a tool center point (TCP) point of a robotic arm 410 (i.e. a midpoint of the line connecting the radiation source 430 and the detector 440). As shown in FIG. 4, the X-ray imaging device 400 is a digital subtraction angiography (DSA) device.

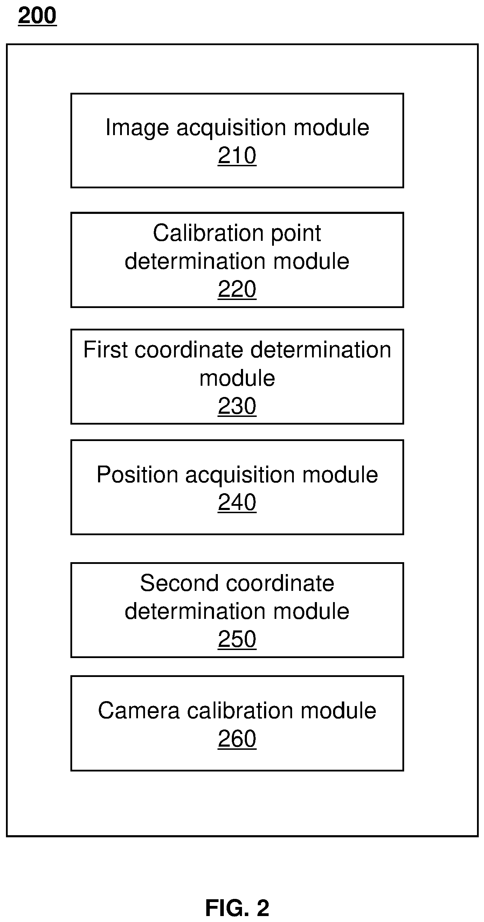

FIG. 2 is a schematic diagram illustrating exemplary camera calibration system for X-ray imaging according to some embodiments of the present disclosure.

As shown in FIG. 2, a camera calibration system 200 for X-ray imaging may include an image acquisition module 210, a calibration point determination module 220, a first coordinate determination module 230, a position acquisition module 240, a second coordinate determination module 250, and a camera calibration module 260. In some embodiments, modules of the camera calibration system 200 for X-ray imaging may be implemented by the processing device 120.

In some embodiments, the image acquisition module 210 may be configured to acquire at least one image taken by a camera to be calibrated, wherein the at least one image may include a calibration target, and the calibration target may include at least one calibration point.

In some embodiments, the calibration point determination module 220 may be configured to select any calibration point of the at least one calibration point as a target calibration point.

In some embodiments, the calibration point determination module 220 may be configured to move the calibration target to make the target calibration point at a third position, and designate the target calibration point located as the third position as a target calibration point in subsequent operations.

In some embodiments, the calibration point determination module 220 may be configured to select another calibration point of the at least one calibration point as a target calibration point in subsequent operations.

In some embodiments, the first coordinate determination module 230 may be configured to determine image coordinates of the target calibration point based on the at least one image.

In some embodiments, the position acquisition module 240 may be configured to a first position and a second position of an X-ray imaging device, wherein when the X-ray imaging device is located in the first position and the second position, the target calibration point is within an imaging field of view of the X-ray imaging device, and a first line and a second line are not parallel, the first line connecting a radiation source and a detector of the X-ray imaging device at the first position, the second line connecting the radiation source and the detector of the X-ray imaging device at the second position.

In some embodiments, when the X-ray imaging device is located in the first position and the second position, a line connecting the radiation source and the detector may coincide with a geometric center of the target calibration point.

In some embodiments, the first position may be a position where the line connecting the radiation source and the detector is perpendicular to a plane of the calibration target, and the second position may be a position where the line connecting the radiation source and the receiving is parallel to the plane of the calibration target.

In some embodiments, the position acquisition module 240 may be configured to obtain the first position by the following operations: acquiring a first image captured by the X-ray imaging device at a first candidate position, the first image including the target calibration point; determining a first magnification based on the first image; determining a first distance between a first image position of the target calibration point in the first image and an image center of the first image; determining, based on the first magnification and the first distance, a second distance between a spatial position of the target calibration point and the first line; determining the first position based on the second distance.

In some embodiments, the position acquisition module 240 may be configured to obtain the second position by the following operations: acquiring a second image captured by the X-ray imaging device at a second candidate position, the second image including the target calibration point; determining a second magnification based on the second image; determining a third distance between a second image position of the target calibration point in the second image and an image center of the second image; determining, based on the second magnification and the third distance, a fourth distance between the spatial position of the target calibration point and the second line; determining the second position based on the fourth distance.

In some embodiments, the second coordinate determination module 250 may be configured to determine, based on the first position and the second position, the spatial coordinates of the target calibration point.

In some embodiments, the second coordinate determination module 250 may be configured to determine, based on the first position, first two-dimensional (2D) coordinates of the target calibration point within the plane of the calibration target; determine, based on the second position, second 2D coordinates of the target calibration point within a plane perpendicular to the plane of the calibration target; determine, based on the first 2D coordinates and the second 2D coordinates, the spatial coordinates.

In some embodiments, the first position may correspond to a first angle, and the second position may correspond to a second angle. The second coordinate determination module 250 may be configured to determine first coordinates of the line connecting the radiation source and detector of the X-ray imaging device at the first position based on the first position; determine second coordinates of the line connecting the radiation source and the detector of the X-ray imaging device at the second position based on the second position; determine the spatial coordinates based on the first coordinates and the second coordinates.

In some embodiments, when the X-ray imaging device is located at the first position and/or the second position, the target calibration point may be located at any position within an imaging field of view of the X-ray imaging device.

When the target calibration point is located at any position within the imaging field of view of the X-ray imaging device, the position acquisition module 240 may be configured to obtain spatial coordinates by the following operations: acquiring a first image captured by the X-ray imaging device at the first position, the first image including the target calibration point; determining a first magnification based on the first image; determining a first distance between a first image position of the target calibration point in the first image and an image center of the first image; determining, based on the first magnification and the first distance, a second distance between a spatial position of the target calibration point and the first line; determining first coordinates based on the second distance and the first position; acquiring a second image captured by the X-ray imaging device at the second position, the second image including the target calibration point; determining a second magnification based on the second image; determining a third distance between a second image position of the target calibration point in the second image and the image center of the second image; determining, based on the second magnification and the third distance, a fourth distance between the spatial position of the target calibration point and the second line; determining second coordinates based on the fourth distance and the second position; determining the spatial coordinates based on the first coordinates and the second coordinates.

When the target calibration point is located at any position within the imaging field of view of the X-ray imaging device, the position acquisition module 240 may be configured to obtain the spatial coordinates based on the following operations: acquiring a first image captured by the X-ray imaging device at the first position, the first image including the target calibration point; obtaining first spatial coordinates of the radiation source at the first position; determining second spatial coordinates of a first image position of the target calibration point in the first image; determining, based on the first spatial coordinates and the second spatial coordinates, a first spatial position of a line segment between the radiation source and the first image position; acquiring a second image captured by the X-ray imaging device at the second position, the second image including the target calibration point; obtaining third spatial coordinates of the radiation source at the second position; determining fourth spatial coordinates of a second image position of the target calibration point in the second image; determining, based on the third spatial coordinates and the fourth spatial coordinates, a second spatial position of a line segment between the radiation source and the second image position; determining the spatial coordinates based on the first spatial position and the second spatial position.

In some embodiments, the camera calibration module 260 may be configured to perform calibration on the camera to be calibrated based on the image coordinates and the spatial coordinates.

In some embodiments, the camera calibration module 260 may be configured to determine, based on the image coordinates and the spatial coordinates, a transformation relationship between an image coordinate system of the camera to be calibrated and a spatial coordinate system.

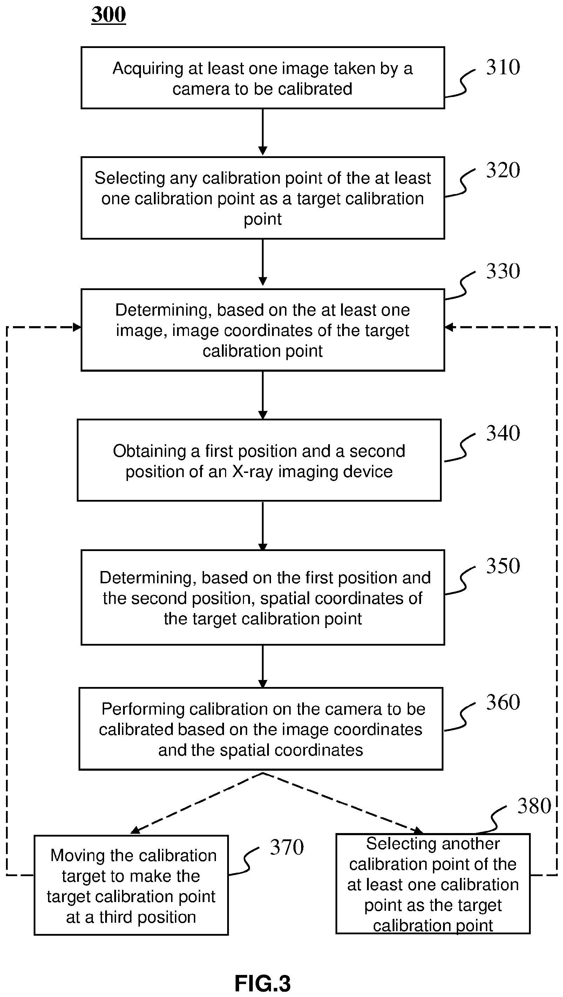

FIG. 3 is a flowchart illustrating an exemplary camera calibration process for X-ray imaging according to some embodiments of the present disclosure.

As shown in FIG. 3, the process 300 may include the following operations. During the X-ray imaging, in order to achieve spatial positioning of the target object, a transformation relationship between the image coordinate system of the camera to be calibrated and the spatial coordinate system needs to be determined, so that the image coordinates of the target object in the camera to be calibrated can be converted to the spatial three-dimension coordinates. In some embodiments, the processing device 120 may perform camera calibration by executing the process 300.

In 310, at least one image taken by a camera to be calibrated may be acquired. The at least one image may include a calibration target, and the calibration target may include at least one calibration point. In some embodiments, the operation 310 may be performed by the image acquisition module 210.

The camera to be calibrated refers to a camera to be calibrated (e.g., the camera 160) for parameters in a system (e.g., the system 100), and the camera to be calibrated may include an optical imaging camera externally installed in various X-ray imaging devices, such as an external camera in the medical imaging device 110, an external camera in the X-ray imaging device 400, or the like. The parameters of the camera to be calibrated may include various parameters related to the camera to be calibrated, such as at least one of internal parameters of the camera to be calibrated (i.e. the transformation relationship between the camera coordinate system of the camera to be calibrated and the image coordinate system of the camera to be calibrated), external parameters (i.e. the conversion relationship between the spatial coordinate system of the camera to be calibrated and the camera coordinate system of the camera to be calibrated), and camera distortion parameters. The camera coordinate system of the camera to be calibrated is a coordinate system based on a position of the camera to be calibrated. The image coordinate system of the camera to be calibrated is a coordinate system established based on a point on the image (e.g., any one of four vertices on a rectangular image or a center point of the image). The spatial coordinate system is a coordinate system established based on any point in space.

The calibration target may be a tool used for camera calibration, which may include various forms, such as chessboard, circular array targets, or standard ball array targets. The calibration point is a preset point on the calibration target used for camera positioning, which may include one or more. In some embodiments, the at least one image may include an image, the processing device 120 may cause the camera to be calibrated to capture any calibration point on the calibration target (e.g. a calibration target 510, a calibration target 700, a calibration target 810, a calibration target 900) by issuing the instructions or other means, thereby obtaining the image including at least a portion of the calibration target. The calibration target in the image may include at least one calibration point.

In some embodiments, the processing device 120 may also obtain the image through other means, such as obtaining from a storage device (e.g., the storage device 130).

In 320, any calibration point of the at least one calibration point may be selected as a target calibration point. In some embodiments, the operation 320 may be performed by the calibration point determination module 220.

In some embodiments, the processing device 120 may choose any calibration point of the at least one calibration point as a target calibration point from the calibration points in the image captured by the camera to be calibrated. The target calibration point may be a calibration point configured to calibrate the camera to be calibrated by performing the following operations.

In 330, image coordinates of the target calibration point may be determined based on the at least one image. In some embodiments, the operation 330 may be performed by the first coordinate determination module 230.

In some embodiments, after determining the target calibration point, the processing device 120 may perform image identification on the target calibration point in the image, thereby determining the image coordinates (also refer to as pixel coordinates) of the target calibration point in the image coordinate system (also refer to as pixel coordinates).

In 340, a first position and a second position of the X-ray imaging device may be obtained. When the X-ray imaging device is located in the first position and the second position, the target calibration point may be within an imaging field of view of the X-ray imaging device. A first line and a second line are not parallel. The first line connects a radiation source and a detector of the X-ray imaging device in the first position, and the second line connects the radiation source and the detector of the X-ray imaging device in the second position. In some embodiments, operation 340 may be performed by the position acquisition module 240. In some embodiments, the processing device 120 may obtain the first position and the second position of the X-ray imaging device. The first position and the second position are not the same. In some embodiments, the first position and the second position may correspond to a position of the camera to be calibrated when the camera captures, and the processing device 120 may determine the first position and the second position based on a target calibration point in an image captured by the camera to be calibrated. Specifically, the processing device 120 may fix a position of the calibration target, then move the X-ray imaging device to the first position and second positions respectively by moving the robotic arm, and capture the image by the camera to be calibrated.

In some embodiments, a position of the X-ray imaging device may be related to a spatial position of a detector and/or the radiation source, an angle of a line connecting the detector and the radiation source (e.g., an angle to a vertical line), a spatial position of the line connecting the detector and the radiation source, a spatial position of a center point of the line connecting the detector and the radiation source, etc.

In some embodiments, the position of the X-ray imaging device (e.g., the first position, the second position, etc.) may be represented by coordinates under a world coordinate system. The world coordinate system is a coordinate system that is independent of the X-ray imaging device and the calibration target, etc., and does not change as the position of the X-ray imaging device and the calibration target, etc., changes. Therefore, changes in coordinates of the X-ray imaging device and the calibration target, etc., under the world coordinate system may reflect changes in their positions. For example, the world coordinate system may be a right-angled coordinate system established with any fixed point in a room in which the X-ray imaging device and the calibration target are placed as an origin, e.g., an X-axis may be a left-right direction, a Y-axis may be a front-back direction, and a Z-axis may be a vertical direction.

In some embodiments, when the X-ray imaging device is located in the first position and the second position, the target calibration point may be within the imaging field of view of the X-ray imaging device. The first line and the second line are not parallel. The first line connects the radiation source and the detector of the X-ray imaging device in the first position, the second line connects the radiation source and the detector of the X-ray imaging device in the second position. An angle between the first line and the second line may be greater than 0 degrees and less than 180 degrees. For example, the angle may be any one of 30°, 45°, 60°, 90°, 120°, or the like. In some embodiments, a line connecting geometric centers of the radiation source and the detector is referred to as a centerline of the X-ray imaging device. The line connecting the radiation source and the detector may overlap with the centerline of the X-ray imaging device. In the present disclosure, the line connecting the radiation source and the detector is the line connecting geometric centers of the radiation source and the detector.

In 350, spatial coordinates of the target calibration point may be determined based on the first position and the second position. In some embodiments, operation 350 may be performed by the second coordinate determination module 250.

The spatial coordinates of the target calibration point are coordinates of the target calibration point in a spatial coordinate system (3D coordinate system), such as coordinates in the form of (X, Y, Z), or the like. In some embodiments, the spatial coordinate system may include a three-dimensional coordinate system based on a variety of references, for example, a world coordinate system, a robotic arm coordinate system, a camera coordinate system, or the like. In some embodiments, the processing device 120 may determine the spatial coordinates of the target calibration point based on the first position and the second position.

The present disclosure provides an embodiment for determining the spatial coordinates of the target calibration point. In the embodiment, when the X-ray imaging device is located in the first position and the second position, the line connecting the radiation source and the detector may overlap with the geometric center of the target calibration point, the first position may be a position where the line connecting the radiation source and the detector is perpendicular to the plane of the calibration target, e.g., an anteroposterior position, and the second position may be a position where the line connecting the radiation source and the detector is parallel to the plane of the calibration target, e.g., a lateral position.

In some embodiments, the processing device 120 may determine first two-dimensional (2D) coordinates of the target calibration point within the plane of the calibration target based on the first position; the processing device 120 may determine second 2D coordinates of the target calibration point within a plane perpendicular to the plane of the calibration target based on the second position; and the processing device 120 may determine the spatial coordinates based on the first 2D coordinates and the second 2D coordinates.

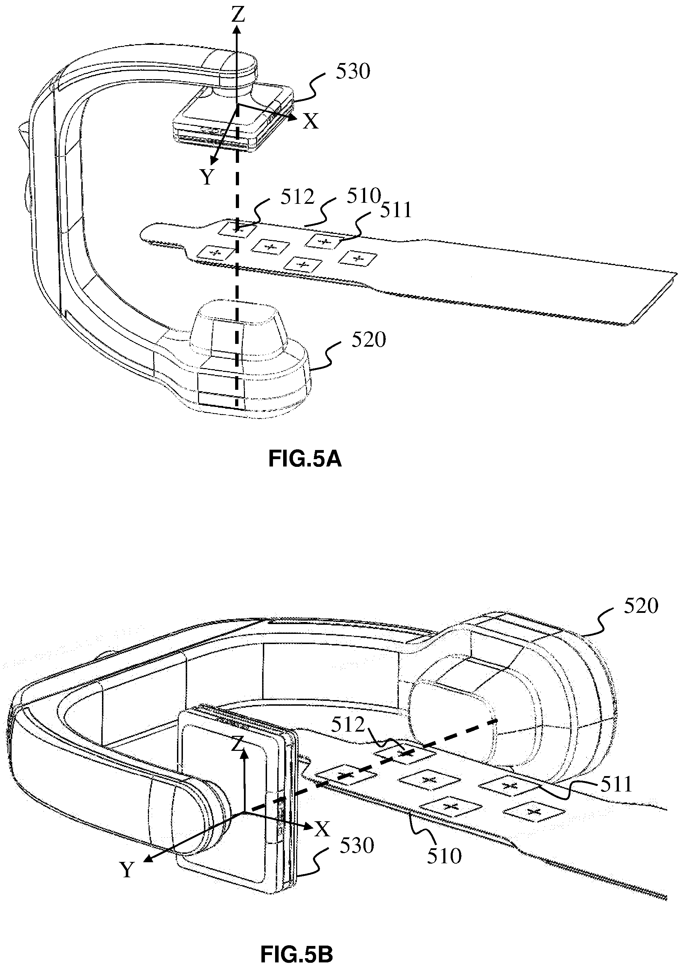

More descriptions of determining the spatial coordinates of the target calibration point based on the first position and the second position may be found in FIG. 5A, FIG. 5B, and related descriptions. An X-ray imaging device in FIG. 5A is in the first position and the X-ray imaging device in FIG. 5B is in the second position. As shown in FIG. 5A and FIG. 5B, 510 represents a calibration target, 511 represents a calibration unit on the calibration target, 512 represents a target calibration point, and 520 and 530 represent a radiation source and a detector of a robotic arm of the X-ray imaging device. When the X-ray imaging device is located in the first position and the second position, the target calibration point overlaps with the centerline, but there is no way to know where the calibration point is located on the centerline. In some embodiments, the processing device 120 may record coordinates of a TCP in real-time by an X-ray imaging device.

As shown in FIG. 5A, when the X-ray imaging device is in the first position, a line connecting a radiation source 520 and a detector 530 of the robotic arm is perpendicular to a plane of the calibration target 510, and the line overlaps with the target calibration point 512. Therefore, the target calibration point 512 has the same two-dimensional planar coordinates as the TCP, so that coordinates of the target calibration point within the plane (i.e., a two-dimensional horizontal plane) of the calibration target 510 may be obtained, e.g., X-coordinates and Y-coordinates as shown in FIG. 5A, which represents that a horizontal position of the target calibration point relative to the camera of the X-ray imaging device is determined.

As shown in FIG. 5B, when the X-ray imaging device is in the second position, the line connecting the radiation source 520 and the detector 530 of the robotic arm is parallel to the plane of the calibration target 510, and a center point of the line overlaps with the target calibration point 512. Thus, the target calibration point 512 has the same two-dimensional planar coordinates as the TCP, and coordinates of the target calibration point within a plane (i.e., a two-dimensional vertical plane) perpendicular to the plane of the calibration target 510 may be obtained, for example, X-coordinates and Z-coordinates as shown in FIG. 5B. By combining the coordinates of the target calibration point within the horizontal plane and within the vertical plane, the coordinates of the target calibration point in a three-dimensional space (X-coordinates, Y-coordinates, and Z-coordinates) may be determined, which represents that a vertical position of the target calibration point relative to the camera of the X-ray imaging device is determined.

In 360, calibration may be performed on the camera to be calibrated based on the image coordinates and the spatial coordinates. In some embodiments, operation 360 may be performed by the camera calibration module 260.

In some embodiments, the processing device 120 may calibrate the camera to be calibrated based on the image coordinates and the spatial coordinates, i.e., determine parameters associated with the camera to be calibrated, e.g., at least one of a transformation relationship between an image coordinate system and a spatial coordinate system of the camera to be calibrated, an internal camera reference, an external camera reference, and a camera distortion parameter, or the like. In some embodiments, the internal camera reference may be known and the external camera reference may be determined based on the internal camera reference, the image coordinates, and the spatial coordinates.

In some embodiments, the processing device 120 may, based on the image coordinates (2D coordinates) and the spatial coordinates (3D coordinates) of the target calibration point, determine a transformation relationship between the camera coordinate system and the spatial coordinate system of the camera to be calibrated by a matrix transformation of the coordinates, i.e., a transformation matrix of a same point in the camera coordinate system and the spatial coordinate system.

In some embodiments, the processing device 120 may determine at least one of the internal camera reference, the external camera reference, the camera distortion parameter, etc. based on the image coordinates and spatial coordinates of the target calibration point. In some embodiments, based on image coordinates and spatial coordinates of a plurality of target calibration points, positional deviations of the plurality of target calibration points on an image may be determined, and camera distortion parameters may be determined based on the positional deviations. In some embodiments, the internal camera reference is known, the processing device 120 may determine the coordinates in the camera coordinate system based on the image coordinates and the internal camera reference, and then determine the external camera reference based on the coordinates in the camera coordinate system and the spatial coordinates in the spatial coordinate system.

In some embodiments, after operation 360, the processing device 120 may switch a new position of a target calibration point by performing operation 370 or operation 380, and then re-perform operations 330 to 360 based on the new position of the target calibration point to perform calibration on the camera to be calibrated again. The operation may be looped multiple times to obtain a plurality of calibration results for the camera to be calibrated. Each of the calibration results includes at least one parameter associated with the camera to be calibrated, and the parameters included in the plurality of calibration results are of the same type. For example, each of the plurality of calibration results includes a transformation relationship between an image coordinate system and a spatial coordinate system of the camera to be calibrated. For example, each of the plurality of calibration results includes an external camera reference.

In some embodiments, when the plurality of calibration results for the camera to be calibrated are obtained, the processing device 120 may determine a final calibration result based on the plurality of calibration results. For example, a calibration result with a highest frequency of occurrence may be determined as the final calibration result.

In 370, the calibration target may be moved to make the target calibration point located at a third position. In some embodiments, the operation 370 may be performed by the calibration point determination module 220.

In some embodiments, the processing device 120 may move the calibration target so that the target calibration point is in a different position from a previous position, i.e., the third position, thus changing the spatial position of the line connecting the radiation source and the detector relative to the calibration target point.

In some embodiments, after making the target calibration point at the third position, the processing device 120 may continue to perform operations between operations 330-360 based on a position of the new target calibration point.

In 380, another calibration point of the at least one calibration point may be selected as the target calibration point. In some embodiments, the operation 380 may be performed by the calibration point determination module 220.

In some embodiments, the processing device 120 may select another calibration point among the plurality of calibration points of the calibration target as a new target calibration point, wherein this new target calibration point is different from an original target calibration point, thereby changing the spatial position of the line connecting the radiation source and the detector relative to the target calibration point.

In some embodiments, after selecting another calibration point as a new target calibration point, the processing device 120 may continue to perform the operations between operations 330-360 based on a position of the new target calibration point.

In some embodiments of the present disclosure, by capturing images of a same calibration point by the camera of the X-ray imaging device at different positions and determining the spatial position of the calibration point based on the relative position between the calibration point and the centerline of the X-ray imaging device, spatial three-dimensional coordinates of the calibration point are obtained through the images directly, which avoids the problem of a low coordinate accuracy due to using a ruler to measure the calibration point when using a large calibration target; and when measuring a plurality of calibration points, the same reference (the same position of the X-ray imaging device) may not result in the relative measurement error between the calibration points, which improves the measurement accuracy and the measurement speed. At the same time, by overlapping the line connecting the radiation source and the detector with the calibration point, the position of the calibration point is indirectly obtained through the real-time feedback of the position of the TCP, which improves the measurement accuracy.

In some embodiments, the processing device 120 may acquire a target image taken by the calibrated camera, wherein the image includes an object. The processing device 120 may determine image coordinates of the object in an image coordinate system of the target image (e.g., image coordinates of a center point of the object in the target image). The processing device 120 may determine spatial coordinates of the object (e.g., spatial coordinates of a center point of the object) in a spatial coordinate system based on the image coordinates of the object and a calibration result (e.g., a transformation relationship between the image coordinate system of the camera and the spatial coordinate system) of the calibrated camera. The processing device 120 may move, based on the spatial coordinates of the object, the X-ray imaging device to a third position at which a center point of a third line connecting the radiation source and the detector of the X-ray imaging device overlaps the object (e.g., a spatial coordinates of the center point of the third line in the spatial coordinate system coincides with the spatial coordinates of the object). The processing device 120 may cause the X-ray imaging device to scan the object at the third position.

For operation 340, the present disclosure provides an embodiment for obtaining the first position and the second position of an X-ray imaging device.

In some embodiments, when the X-ray imaging device is located in the first position and/or the second position, the line connecting the radiation source and the detector overlaps with a geometric center of the target calibration point, i.e., the line passes through the geometric center of the target calibration point. In some embodiments, the processing device 120 may calculate a positional deviation between the line connecting the radiation source and the detector (e.g., a center point of the line (i.e., a TCP)) and the geometric center of the target calibration point, move the robotic arm according to the deviation (e.g., adjust by real-time perspective and low-speed motion, etc.), so as to cause the line connecting the radiation source and the detector to coincide with the center of the target calibration point, at which point a position of the X-ray imaging device may be taken as the first position and/or the second position.

In some embodiments, the processing device 120 may perform real-time perspective by the X-ray imaging device, and then manually adjust the position of the robotic arm (e.g., robotic arm 410) by a user, so that the line connecting the radiation source and the detector coincides with the center of the target calibration point.

In some embodiments, the processing device 120 may determine the first position by the following operations: acquiring a first image including the target calibration point captured by the X-ray imaging device in a first candidate position; determining a first magnification based on the first image; determining a first deviation between the target calibration point and an image center of the first image based on the first image; determining a second deviation between a spatial position of the target calibration point and the centerline of the X-ray imaging device based on the first magnification and the first deviation; and determining the first position based on the second deviation.

In some embodiments, the processing device 120 may determine the first candidate position based on a predetermined condition. The predetermined condition may be that when the X-ray imaging device is located in the first candidate position and the first position, respectively, lines connecting the radiation source and the detector of the X-ray imaging device are at an equal angle in space, and the target calibration point is located within the imaging field of view. In some embodiments, when the X-ray imaging device is located in the first candidate position and the first position, respectively, coordinates of the lines connecting the radiation source and the detector in space may be the same (i.e., the two lines overlap), or may be different (i.e., the two lines are parallel).

In some embodiments, after determining the first candidate position, the processing device 120 may acquire the first image including the target calibration point captured by the X-ray imaging device in the first candidate position. In some embodiments, the processing device 120 may determine, based on whether the target calibration point in the first image is located in the image center, whether the coordinates of the lines connecting the radiation source and the detector of the X-ray imaging device in space are the same (whether the two lines overlap) when the X-ray imaging device is located in the first candidate position and the first position, respectively. When the target calibration point is located at the image center of the first image, the two lines overlap; otherwise, the two lines are parallel, and the processing device 120 may determine the first position based on a deviation between the two lines.

In some embodiments, the processing device 120 may determine a proportion relationship between a size of an object (at least a portion of the calibration target) in the first image and an actual size of the object, and determine the proportion relationship as the first magnification. For example, a ratio of a size of the object in the first image to an actual size of the object may be any of 1:100, 1:200, 1:1000, or the like.

In some embodiments, the processing device 120 may determine a deviation between a position of the target calibration point in the first image and the image center of the first image as a first deviation. The first deviation includes a distance and/or direction of the position of the target calibration point in the first image from the image center of the first image. The centerline of the X-ray imaging device at the time of capture passes through an image center of a captured image (e.g., the first image, a second image, etc.).

In some embodiments, the processing device 120 may determine, based on the first magnification, a spatial actual deviation corresponding to the first deviation, and determine the actual deviation as a deviation (distance and/or direction) between the spatial position of the target calibration point and the centerline of the X-ray imaging device in an actual space, i.e., a second deviation. For example, when a distance between the position of the target calibration point in the first image and the image center of the first image is 1 mm, and the first magnification is 1:100, a distance between the target calibration point and the centerline of the X-ray imaging device in the actual space is 1*100 = 100 mm.

In some embodiments, the processing device 120 may determine the first position as a position that has the second deviation in distance and/or direction from the first candidate position. In some embodiments, the processing device 120 may determine an orientation of the spatial position of the target calibration point with respect to the X-ray imaging device based on an orientation of the position of the target calibration point in the first image with respect to the image center of the first image, and determine the orientation of the spatial position of the target calibration point with respect to the X-ray imaging device as an orientation of the first position with respect to the first candidate position.

In some embodiments, after determining the first position, the processing device 120 may generate movement information of the X-ray imaging device based on the second deviation, then manually or automatically move the X-ray imaging device to the first position based on the movement information.

In some embodiments, after the X-ray imaging device is moved to the first position, the processing device 120 may capture an image again by the X-ray imaging device to check whether the target calibration point in the image is located at a center point of the image. If the target calibration point is not located at the center point of the image, then the above operation of determining the first position is repeated until the target calibration point is located at the center point of the image.

In some embodiments, the processing device 120 may determine the second position by a following manner: acquiring a second image including the target calibration point captured by the X-ray imaging device in a second candidate position; determining a second magnification based on the second image; determining a third deviation between the target calibration point and an image center of the second image based on the second image; determining a fourth deviation between the spatial position of the target calibration point and the centerline of the X-ray imaging device based on the second magnification and the third deviation; and determining the second position based on the fourth deviation.

In some embodiments, a manner of determining the second position may be similar to that of determining the first position, wherein the second image may correspond to the first image, the second candidate position may correspond to the first candidate position, the second magnification may correspond to the first magnification, the third deviation may correspond to the first deviation, and the fourth deviation may correspond to the second deviation. In some embodiments, the first candidate position may be different from the second candidate position.

The processing device 120 may perform the above operations to determine a first position and/or a second position in which the line connecting the radiation source and the detector overlaps with a geometric center of the target calibration point.

The present disclosure also provides another process for determining the first magnification and the second magnification. The process may include acquiring a third image captured by the X-ray imaging device at a first auxiliary position; obtaining first coordinates related to the X-ray imaging device at the first candidate position; obtaining second coordinates related to related to the X-ray imaging device at the first auxiliary position; obtaining a third coordinates of the target calibration point in the first image; obtaining a fourth coordinates of the target calibration point in the third image; and determining the first magnification based on the first coordinates, the second coordinates, the third coordinates, and the fourth coordinates. The third image includes the target calibration point.

In some embodiments, a line connecting the radiation source and the detector at the first candidate position and a line connecting the radiation source and the detector at the first auxiliary position may be parallel.

In some embodiments, the first coordinates and the second coordinates are the coordinates of the radiation source, the detector, or a center point of a line connecting the radiation source and the detector at the first candidate position and the first auxiliary position, respectively.

In some embodiments, the first coordinates and the second coordinates may be in a same plane parallel to a plane of the calibration target. For example, the X-ray imaging device performs a translational movement in the plane of the calibration target so as to obtain the first candidate position and the first auxiliary position.

In some embodiments, the line connecting the radiation source and the detector at the first candidate position and the line connecting the radiation source and the detector at the first auxiliary position are at an angle (e.g., [0°,90°) with a line vertical to a plane of the calibration target.

In some embodiments, the third coordinates and the fourth coordinates may be two-dimensional (2D) coordinates in a first coordinate system related to the detector. The first coordinates and the second coordinates may be two-dimensional (2D) coordinates in a second coordinate system related to the plane of the calibration target. When the line connecting the radiation source and the detector at the first candidate position and the line connecting the radiation source and the detector at the first auxiliary position are at an angle of 0°with the line vertical to the plane of the calibration target (i.e., the detector is parallel with the calibration target), the two axis of the first coordinate system may be parallel with the two axis of the second coordinate system. For example, the first coordinates are (x1, y1), the second coordinates are (x2, y2), the third coordinates are (u1, v1), and the fourth coordinates are (u2, v2). The x axis of the first coordinate system is parallel with the u axis of the second coordinate system, and the y axis of the first coordinate system is parallel with the v axis of the second coordinate system. The first magnification may be (x1-x2)/(u1-u2) or (y1-y2)/(v1-v2).

When the line connecting the radiation source and the detector at the first candidate position and the line connecting the radiation source and the detector at the first auxiliary position are at an angle ( θ ≠ 0°) with the line vertical to the plane of the calibration target (i.e., the detector is not parallel with the calibration target), the first magnification may be determined based further on the angle. For example, the first magnification may be determined based on (x1-x2)/(u1-u2) and the angle θ, or (y1-y2)/(v1-v2) and the angle θ.

The process for determining the second magnification may be similar to the process for determining the first magnification. The process for determining the second magnification may include acquiring a fourth image captured by the X-ray imaging device at a second auxiliary position; obtaining fifth coordinates related to the X-ray imaging device at the second candidate position; obtaining sixth coordinates related to related to the X-ray imaging device at the second auxiliary position; obtaining a seventh coordinates of the target calibration point in the second image; obtaining an eighth coordinates of the target calibration point in the fourth image; and determining the second magnification based on the fifth coordinates, the sixth coordinates, the seventh coordinates, and the eighth coordinates. The fourth image includes the target calibration point.

In some embodiments, the first magnification may be equal to the second magnification. In this case, the process for determining the second magnification may be omitted.

For operation 350, the present disclosure provides another embodiment for determining the spatial coordinates of the target calibration point. In the embodiment, when the X-ray imaging device is located in the first position and the second position, the line connecting the radiation source and the detector may overlap with the geometric center of the target calibration point, the first position may correspond to a first angle, and the second position may correspond to a second angle. The first angle and the second angle may be an angle of the line connecting the radiation source and the detector relative to a reference plane. The reference plane may be a predetermined plane with relatively fixed positions, for example, one of a calibration target plane, a horizontal plane, a vertical plane, or the like. In some embodiments, an absolute value of a difference between the first angle and the second angle may be any value greater than 0 degrees and less than 180 degrees, i.e., the lines connecting the radiation source and the detector are not parallel (e.g., the difference between the first angle and the second angle may be 90 degrees) when the X-ray imaging device is located in the first position and the second position, respectively.

The following of the present disclosure are all illustrated as an example where the reference plane is the horizontal plane. At this point, the processing device 120 may take an angle of the line connecting the radiation source and the detector relative to the calibration target plane when the X-ray imaging device is in the first position as the first angle; and take an angle of the line connecting the radiation source and the detector relative to the horizontal plane when the X-ray imaging device is in the second position as the second angle.

In some embodiments, the processing device 120 may determine, based on the first position, first coordinates of the line connecting the radiation source and the detector of the X-ray imaging device in the first position; based on the second position, the processing device 120 may determine second coordinates of the line connecting the radiation source and the detector of the X-ray imaging device in the second position; and based on the first coordinates and the second coordinates, the processing device 120 may determine the spatial coordinates. The first coordinates and the second coordinates may be three-dimensional coordinates.

In some embodiments, when the X-ray imaging device is in the first position, an angle between the line connecting the radiation source and the detector and a plane of the calibration target is the first angle, and the line overlaps with the target calibration point. At this time, the target calibration point is located at the image center of the image captured by the X-ray imaging device in the first position, and since coordinates of the radiation source and the detector are known, spatial coordinates of the line connecting the radiation source and the detector may be determined based on the coordinates of the radiation source and the detector, and the spatial coordinates of the line may be determined as the first coordinates.

In some embodiments, when the X-ray imaging device is in the second position, an angle between the line connecting the radiation source and the detector and the plane of the calibration target is the second angle, and the line overlaps with the target calibration point. At this time, the target calibration point is located at the image center of the image captured by the X-ray imaging device in the second position, and spatial coordinates of the line connecting the radiation source and the detector may be determined based on the coordinates of the radiation source and the detector, and the spatial coordinates of the line may be determined as the second coordinates.

In some embodiments, the processing device 120 may determine the spatial coordinates of the target calibration point based on the first coordinates and the second coordinates. Specifically, when the X-ray imaging device is in the first position and the second position, respectively, the lines connecting the radiation source and the detector both pass through the target calibration point. Thus, if there is an intersection point of the two lines, the processing device 120 may determine coordinates of the intersection point based on the first coordinates and the second coordinates, then the coordinates of the intersection point may be determined as the spatial coordinates of the target calibration point; and if there is no intersection point of the two lines, the processing device 120 may determine coordinates of a center point of a shortest distance between the two lines based on the first coordinates and the second coordinates, and determine the coordinates of the center point as the spatial coordinates of the target calibration point. Specifically, if the shortest distance is less than a preset threshold, the processing device 120 may determine the coordinates of the center point as the spatial coordinates of the target calibration point.

In some embodiments, the target calibration point may be located anywhere within the imaging field of view of the X-ray imaging device when the X-ray imaging device is located in the first position and/or the second position. For example, any of a center of the imaging field of view, an edge position of the imaging field of view, or the like.

The present disclosure provides another embodiment for determining the spatial coordinates of the target calibration point. In the embodiments, the target calibration point is located anywhere within the imaging field of view of the X-ray imaging device.

The processing device 120 may perform the following operations to determine the spatial coordinates of the target calibration point. The operations may include: acquiring a first image captured by the X-ray imaging device at the first position, the first image including the target calibration point; determining a first magnification based on the first image; determining a first deviation between a first image position of the target calibration point in the first image and an image center of the first image; determining, based on the first magnification and the first deviation , a second deviation between a spatial position of the target calibration point and the first line; acquiring a second image captured by the X-ray imaging device at the second position, the second image including the target calibration point; determining a second magnification based on the second image; determining a third deviation between a second image position of the target calibration point in the second image and the image center of the second image; determining, based on the second magnification and the third deviation, a fourth deviation between the spatial position of the target calibration point and the second line; and determining the spatial coordinates based on the second deviation and the fourth deviation.

The processing device 120 may acquire the first image including the target calibration point captured by the X-ray imaging device in the first position, and the second image including the target calibration point captured by the X-ray imaging device in the second position, and determine the spatial coordinates of the target calibration point based on the first image and the second image.

In some embodiments, after determining the first position, the processing device 120 may acquire the first image including the target calibration point captured by the X-ray imaging device in the first position in various ways. For example, the first image may be acquired from images that have been captured by the X-ray imaging device. As another example, it is possible to obtain the first image by having the X-ray imaging device capture the target calibration point in the first position. Acquiring the second image is performed in a manner similar to acquiring the first image, and is not described herein.

In some embodiments, after acquiring the first image, the processing device 120 may determine the second deviation of the spatial position of the target calibration point from the centerline of the X-ray imaging device by determining the first magnification based on the first image; determining the first deviation of the target calibration point from the image center of the first image, the first deviation comprising a distance and/or an direction of the position of the target calibration point on the first image from the image center of the first image; determine the second deviation based on the first magnification and the first deviation.

The manner of determining the first magnification, the first deviation, and the second deviation may be similar to that in operation 320, and may not be repeated herein.

In some embodiments, after acquiring the second image, the processing device 120 may determine the fourth deviation of the spatial position of the target calibration point from the centerline of the X-ray imaging device by acquiring the second image including the target calibration point captured by the X-ray imaging device in the second position; determining the second magnification based on the second image; determining the third deviation of the target calibration point from the image center of the second image based on the second image; and determining the fourth deviation based on the second magnification and the third deviation.

The process of determining the second magnification, the third deviation, and the fourth deviation may be performed in a manner similar to that in operation 320, and may not be repeated here.

In some embodiments, after determining the second deviation and the fourth deviation, the processing device 120 may determine the spatial coordinates based on the second deviation and the fourth deviation.

In some embodiments, the processing device 120 may determine the first 2D coordinates based on the second deviation. Specifically, when in the first position, the line connecting the radiation source and the detector is perpendicular to the plane of the calibration target. When the first position is determined, position coordinates of center point of the line connecting the radiation source and the detector in the first position are also determined. The processing device 120 may determine, based on the position coordinates and the second deviation, coordinates of the center point of the line connecting the radiation source and the detector when the target calibration point and the line connecting the radiation source and the detector overlap, and determine the first 2D coordinates of the target calibration point within the plane of the calibration target based on the coordinates. More descriptions about how to determine the first 2D coordinates may be found in the foregoing, and will not be repeated here.

In some embodiments, the processing device 120 may determine the second 2D coordinates based on the fourth deviation in a manner similar to the manner of determining the first 2D coordinates based on the second deviation, which will not be repeated herein.

In some embodiments, the processing device 120 may determine the spatial coordinates based on the first 2D coordinates and the second 2D coordinates.

In some embodiments, the processing device 120 may determine the first coordinates based on the second deviation. Specifically, when in the first position, the line connecting the radiation source and the detector is not necessarily perpendicular to the plane of the calibration target. When the first position is determined, position coordinates of the radiation source and the detector in the first position are also determined. The processing device 120 may determine, based on the position coordinates and the second deviation, required movement information of the X-ray imaging device when a calibration point overlaps with the centerline; determine coordinates of the radiation source and the detector after movement based on the movement information; determine, based on the coordinates of the radiation source and the detector, the spatial coordinates of the line connecting the radiation source and the detector as the first coordinates. Further details on how to determine the first coordinates may be found in the foregoing, and will not be repeated here.

In some embodiments, the processing device 120 may determine the second coordinates based on the fourth deviation in a manner similar to the manner of determining the first coordinates based on the second deviation, which will not be repeated herein.

In some embodiments, the processing device 120 may determine the spatial coordinates based on the determined first coordinates and second coordinates.

The present disclosure provides another embodiment for determining the spatial coordinates of the target calibration point. In the embodiment, the target calibration point is located anywhere within the imaging field of view of the X-ray imaging device.

The processing device 120 may perform the following operations to determine the spatial coordinates of the target calibration point. The operations may include: acquiring a first image captured by the X-ray imaging device at the first position, the first image including the target calibration point; obtaining first spatial coordinates of the radiation source at the first position; determining second spatial coordinates of a first image position of the target calibration point in the first image; determining, based on the first spatial coordinates and the second spatial coordinates, a first spatial position of a line segment between the radiation source and the first image position; acquiring a second image captured by the X-ray imaging device at the second position, the second image including the target calibration point; obtaining third spatial coordinates of the radiation source at the second position; determining fourth spatial coordinates of a second image position of the target calibration point in the second image; determining, based on the third spatial coordinates and the fourth spatial coordinates, a second spatial position of a line segment between the radiation source and the second image position; and determining the spatial coordinates based on the first spatial position and the second spatial position.

In some embodiments, after acquiring the first image, the processing device 120 may determine a first spatial position of a line segment between the radiation source and a first image position by acquiring the first image including the target calibration point captured by the X-ray imaging device in the first position. The processing device 120 may acquire first spatial coordinates of the radiation source in the first position. The processing device 120 may determine second spatial coordinates of the target calibration point at the first image position in the first image; and determine the first spatial position of the line segment between the radiation source and the first image position based on the first spatial coordinates and the second spatial coordinates.

In some embodiments, when the first position is determined, spatial positions of the radiation source and the detector of the X-ray imaging device, and a spatial position of the centerline of the X-ray imaging device are also determined, and thus, the processing device 120 may determine a spatial position of a center point (shape center) of the radiation source, and determine the center point as the first spatial coordinates. The center point of the radiation source and a center point of the detector (shape center) are located on the centerline of the X-ray imaging device.

The image position is a corresponding point of the target calibration point in an image. The first image position is a corresponding point of the target calibration point in an image captured in the first position. The second image position is a corresponding point of the target calibration point in an image captured in the second position.

In some embodiments, the processing device 120 may determine a spatial coordinate of the first image position, i.e., second spatial coordinates, based on a spatial position of a center of the detector, and a positional deviation of the image center on the first image from the first image position.

In some embodiments, the line segment between the radiation source and the first image position/second image position may be a line segment between the center point of the emitting end and the first image position/second image position. In some embodiments, after determining the first spatial coordinates and the second spatial coordinates, the processing device 120 may determine, based on the first spatial coordinates and the second spatial coordinates, the line segment (also referred to as a first line segment) between the radiation source and the first image position and a spatial position of the line segment, i.e., the first spatial position.

In some embodiments, after acquiring the second image, the processing device 120 may determine a second spatial position of a line segment between the radiation source and a second image position by acquiring third spatial coordinates of the radiation source in the second position; determining fourth spatial coordinates of the target calibration point in the second image position in the second image; determining the second spatial position of the line segment between the radiation source and the second image position based on the third spatial coordinates and the fourth spatial coordinates; and determining the spatial coordinates based on the first spatial position and the second spatial position.

In some embodiments, similar to determining the first spatial coordinates, the processing device 120 may determine the spatial coordinates of the center point (shape center) of the radiation source based on the second position and determine the spatial coordinates as the third spatial coordinates.

In some embodiments, similar to determining the fourth spatial coordinates, the processing device 120 may determine spatial coordinates of the second image position, i.e., the fourth spatial coordinates based on the spatial position of the center of the detector, and a positional deviation of the image center on the second image from the second image position.

In some embodiments, similar to determining the first spatial position, after determining the third spatial coordinates and the fourth spatial coordinates, the processing device 120 may determine, based on the third spatial coordinates and the fourth spatial coordinates, a line segment (also referred to as a second line segment) between the radiation source and the second image position and a spatial position of the line segment, i.e., the second spatial position.

In some embodiments, the processing device 120 may determine the spatial coordinates based on the first spatial position and the second spatial position. Specifically, if there is an intersection point between the first line segment and the second line segment, the intersection point is determined to be the target calibration point, and if there is no intersection point between the first line segment and the second line segment, a center of a shortest distance between the two line segments is determined as the target calibration point. Thereby, the spatial position (i.e., the spatial coordinates) of the target calibration point is determined based on the first spatial position (the spatial position of the first line segment) and the second spatial position (the spatial position of the second line segment).

It should be noted that the foregoing description of the process 300 is intended to be exemplary and illustrative only and does not limit the scope of application of the present disclosure. For a person skilled in the art, various corrections and changes can be made to the process 300 under the guidance of the present disclosure. However, these corrections and changes remain within the scope of the present disclosure. For example, in selecting the target calibration point multiple times, the target calibration point may be selected by alternating between operations 370 and 380, i.e., if the previous time was to select the target calibration point by operation 370, this time, it is possible to select the target calibration point by operation 380.

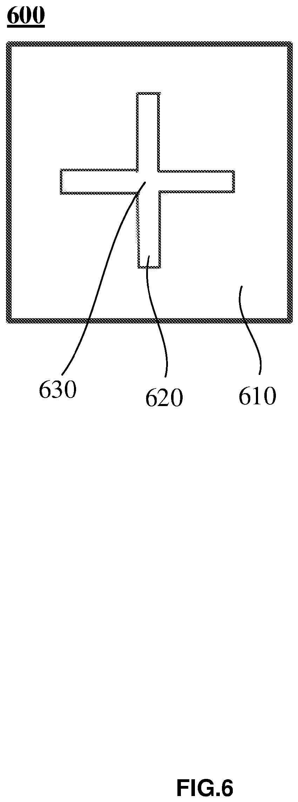

FIG. 6 is a schematic diagram illustrating an exemplary structure of a calibration unit according to some embodiments of the present disclosure.

In some embodiments, a calibration target (e.g., the calibration target 510, a calibration target 700, a calibration target 810, a calibration target 900) may include at least one calibration unit. Each of the calibration units may include a calibration part and a base plate, a geometric center of the calibration part is set as a calibration point, the calibration part is disposed on the base plate, and there is an X-ray attenuation difference and an optical imaging difference between the calibration part and the base plate. The X-ray attenuation difference is used to distinguish different objects (e.g., the calibration part and the base plate are objects of different materials) in an X-ray image. The optical imaging difference is used to distinguish between different objects in an optical image (e.g., the calibration part and the base plate are objects of different shape). The X-ray attenuation difference and the optical imaging difference between the calibration part and the base plate may ensure that the calibration part can be recognized under both X-ray imaging and optical imaging, which improves the calibration accuracy.

As shown in FIG. 6, a calibration unit 600 may include a base plate 610 and a calibration part 620, the calibration part 620 has a geometric center 630, the geometric center 630 is set as the calibration point, the calibration part 620 is disposed on the base plate 610, and there is an X-ray attenuation difference and an optical imaging difference between the calibration part 620 and the base plate 610.

In some embodiments, the calibration part may be geometrically symmetric (i.e., symmetry on both sides, center, etc.). As shown in FIG. 6, the calibration part 620 is centrally symmetrical.

In some embodiments, at least one of a hollow part, a convex part, a concave part, and a splicing part, etc., may be provided around the geometric center of the calibration part (i.e., the calibration point), wherein at least one of the hollow part, a convex part, a concave part, and a splicing part forms the calibration part. For example, the calibration part that is a splicing part indicates that the calibration part having an X-ray attenuation difference and an optical imaging difference from the base plate 610 is connected with the base plate.

In some embodiments, only one of the hollow part, the convex part, the concave part, and the splicing part is provided around the geometric center to form the calibration part. For example, only the hollow part or the concave part is provided around the geometric center. In some embodiments, two or more of the hollow part, the convex part, the concave part, and the splicing part are provided around the geometric center to form the calibration part. For example, the hollow part and the convex part are provided around the geometric center to form the calibration part, wherein the convex part are also disposed around the hollow part.

In some embodiments, at least one of the hollow part, the convex part, the concave part, and the splicing part may be provided symmetrically around the geometric center.

In some embodiments, the calibration part may be a hollow cross.

As shown in FIG. 6, the calibration part 620 is a hollow cross, and the geometric center 630 is located at a cross center point of the cross.

In some embodiments, the calibration part may be in other forms, for example, a metal ball, etc.

In some embodiments of the present disclosure, by adopting the hollow cross, the metal ball, or the like as the calibration part, the structure is simple, the cost is low, and it is also capable of having the X-ray attenuation difference and the optical imaging difference, and the calibration accuracy is high.

In some embodiments, there may be a thickness difference or a material difference between the calibration part and the base plate. This allows the calibration part to have the X-ray attenuation difference from the base plate, and the calibration part may be imaged under X-rays. When the calibration part is in the form of a hollow, convex, concave, or the like, the calibration part may be imaged on an X-ray image and an optical image. The above structure is conducive to the formation of the X-ray attenuation difference, which in turn ensures recognizability under X-ray imaging, improves the calibration accuracy, and is simple to make and low cost.

In some embodiments, the geometric centers of the calibration part may overlap in both optical imaging and X-ray imaging. This is conducive to reducing the complexity of the subsequent processing, improving the processing efficiency, and can reduce the calibration error and improve the calibration accuracy.

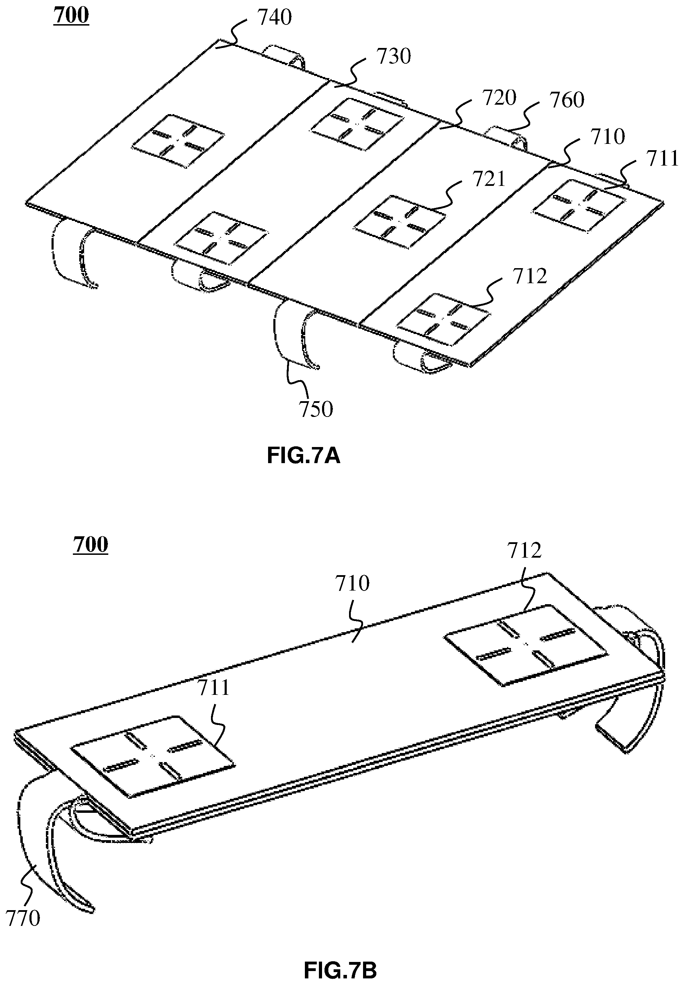

FIG. 7A and FIG. 7B are schematic diagrams illustrating an exemplary a calibration target according to some embodiments of the present disclosure.

In some embodiments, the calibration target may include a support component, the support component may include a support plane, and at least one calibration unit is disposed on the support plane. Each calibration unit on the support plane is recognizable under both X-ray imaging and optical imaging, and because the calibration unit is symmetrical, the calibration accuracy is high. A plurality of calibration units may form a plurality of calibration points, each calibration point is on the same support plane, which is conducive to improving the calibration accuracy under a large field of view, and the production difficulty of the calibration target is low with a low production cost.

As shown in FIG. 7A and FIG. 7B, a calibration target 700 may include a support component, wherein the support component may include support units 710, 720, 730, and 740. The support component has a support plane on which six calibration units (e.g., calibration units 711, 712, 721) are affixed. The calibration unit 711 and an adjacent calibration unit 712 are disposed on the support unit 710, and the calibration unit 721 is disposed on the support unit 720.

In some embodiments, the support component can be folded. The calibration target 700 is in an unfolded state of the support component shown in FIG. 7A, and the calibration target 700 is in a folded state of the support component shown in FIG. 7B. It can be seen that, in the folded state, a volume of the calibration target is greatly reduced, thereby realizing the minimization of a storage space of the calibration target, which is convenient for storing and transporting; at the same time, the size of the calibration target can be flexibly adjusted by folding to adapt to the use of the different fields of view.

As shown in FIG. 7A, the support component is provided with four support units, calibration units (calibration units 711 and 712) are provided at first and last ends of a rightmost support unit 710, respectively, a calibration unit (calibration unit 721) is provided in a middle region of a rightmost adjacent support unit 720, a distribution of calibration units on subsequent two support units is the same as that on the first two support units. This arrangement ensures that calibration units on neighboring support units do not interfere in a process of folding the support component, and ensures that at least one calibration unit is located on an upper surface of the support unit.

In some embodiments, the calibration target may further include a fixing component, wherein one end of the fixing component is connected to the support component. The fixing component may be configured to fix the calibration target, e.g., fix the calibration target to a scanning bed of the X-ray imaging device.

As shown in FIG. 7A and FIG. 7B, the calibration target 700 may include eight fixing components (e.g., fixing components 750, 760, 770), one end of each fixing component is connected to the support component. Each support unit may include two fixing components, e.g., the support unit 720 may include fixing components 750 and 760.

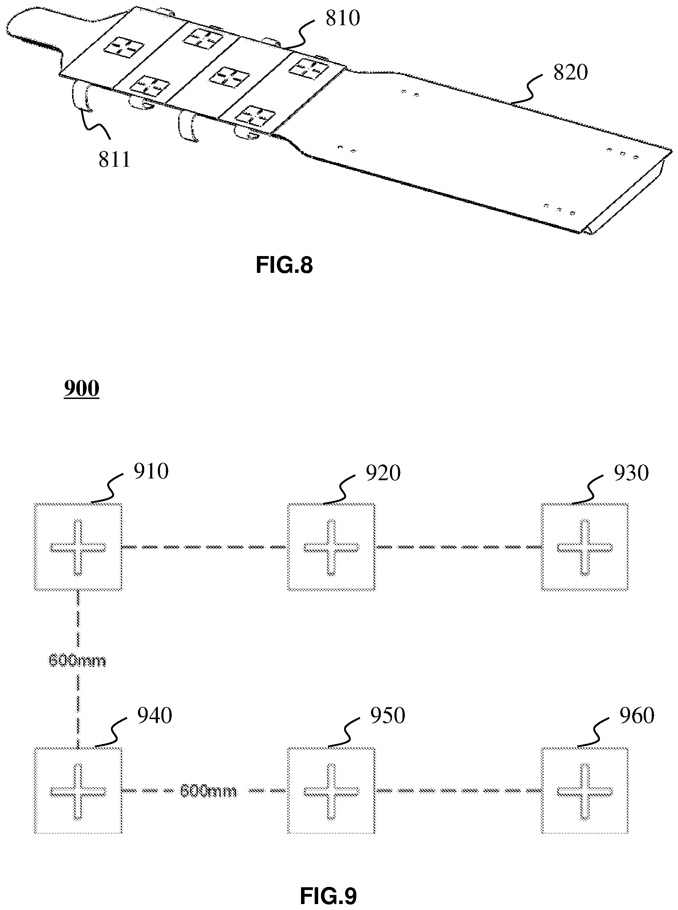

FIG. 8 is a schematic diagram illustrating an exemplary calibration target in use according to some embodiments of the present disclosure.

In some embodiments, the calibration target may be connected to an external component (e.g., a hospital bed, an operating table, etc.) via the fixing component to use the calibration target. This makes it easier to fix the calibration target, and a position of the calibration target is less prone to movement in use, resulting in relatively high calibration accuracy. The fixing component may be in the form of an elastic binding rope, a nylon buckle, or various other forms.

As shown in FIG. 8, a calibration target 810 may be fixed to a hospital bed 820 by the fixing component (e.g., a fixing component 811), and the processing device 120 may use the hospital bed 820 with the fixed calibration target 810 for performing a calibration method for X-ray imaging shown in process 300.

In some embodiments, the calibration target may include at least two calibration points, wherein the at least two calibration points are spaced apart and arranged on an upper surface of a scanning bed of the X-ray imaging device to form at least a portion of the calibration target.

As shown in FIG. 8, the calibration target 810 includes six calibration points that are spaced apart on an upper surface of the hospital bed 820 and form a portion of the calibration target 810.

FIG. 9 is a schematic diagram illustrating an exemplary structure of a calibration target according to some embodiments of the present disclosure.

In some embodiments, the calibration target may include at least two calibration units that are evenly arranged on the support component, which is conducive to improving the calibration accuracy.

As shown in FIG. 9, a calibration target 900 may include six calibration units: calibration units 910, 920, 930, 940, 950, and 960, a bottom of the calibration units is a support component, and the calibration units are uniformly arranged so that a distance between any one calibration unit to a neighboring calibration unit is equal. For example, a distance between the calibration unit 940 and neighboring calibration units 910 and 940 are both 600 mm.

In some embodiments, a spacing distance between neighboring calibration units may be flexibly adjusted as desired. For long-distance and large field of view scenarios, increasing the spacing distance between neighboring calibration units can make the calibration more accurate.

Beneficial effects that may be brought about by the embodiments of the present disclosure include, but are not limited to the followings:(1) by capturing images of the same calibration point by the camera of the X-ray imaging device at different positions and determining the spatial position of the calibration point based on the position of the calibration point relative to the centerline of the X-ray imaging device, the spatial three-dimensional coordinates of the calibration point can be directly obtained through the images, avoiding the problem of a low precision of the coordinates due to using a ruler to measure the calibration point when using a large calibration target; (2) selecting a plurality of calibration points based on a same datum (the same position of the X-ray imaging device) to carry out calibration does not result in a relative measurement error among the plurality of calibration points, and improves the measurement accuracy and the measurement speed; (3) by overlapping the line connecting the radiation source and the detector with the calibration point, the position of the calibration point is indirectly obtained through real-time feedback of the position of the TCP, thereby improving the measurement accuracy; (4) by having the geometrically-centered and symmetrical calibration part, and the X-ray attenuation difference and the optical imaging difference between the calibration part and the base plate, it is ensured that the calibration part can be recognized under both X-ray imaging and optical imaging, thereby improves calibration accuracy. It should be noted that the beneficial effects that may be produced by different embodiments are different, and the beneficial effects that may be produced in different embodiments may be a combination of any one or more of the above, or any other beneficial effect that may be obtained.

The basic concepts have been described. Obviously, for those skilled in the art, the detailed disclosure may be only an example and may not constitute a limitation to the present disclosure. Although not explicitly stated here, those skilled in the art may make various modifications, improvements, and amendments to the present disclosure. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of the specification are not necessarily all referring to the same embodiment. In addition, some features, structures, or features in the present disclosure of one or more embodiments may be appropriately combined.

Moreover, unless otherwise specified in the claims, the sequence of the processing elements and sequences of the present application, the use of digital letters, or other names are not used to define the order of the application flow and methods. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various assemblies described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure and aiding in the understanding of one or more of the various embodiments. However, this disclosure may not mean that the present disclosure subject requires more features than the features mentioned in the claims. In fact, the features of the embodiments are less than all of the features of the individual embodiments disclosed above.

In some embodiments, the numbers expressing quantities, properties, and so forth, used to describe and claim certain embodiments of the application are to be understood as being modified in some instances by the term "about," "approximate," or "substantially." Unless otherwise stated, "about," "approximate," or "substantially" may indicate a ±20% variation of the value it describes. Accordingly, in some embodiments, the numerical parameters set forth in the description and attached claims are approximations that may vary depending upon the desired properties sought to be obtained by a particular embodiment. In some embodiments, the numerical parameters should be construed in light of the number of reported significant digits and by applying ordinary rounding techniques. Although the numerical domains and parameters used in the present application are used to confirm the range of ranges, the settings of this type are as accurate in the feasible range in the feasible range in the specific embodiments.

In addition to the application history documents that are inconsistent or conflicting with the contents of the present disclosure, the documents that may limit the widest range of the claim of the present disclosure (currently or later attached to this application) are excluded from the present disclosure. It should be noted that if the description, definition, and/or terms used in the appended application of the present disclosure is inconsistent or conflicting with the content described in the present disclosure, the use of the description, definition and/or terms of the present disclosure shall prevail.

At last, it should be understood that the embodiments described in the disclosure are used only to illustrate the principles of the embodiments of this application. Other modifications may be within the scope of the present disclosure. Thus, by way of example, but not of limitation, alternative configurations of the embodiments of the present disclosure may be utilized in accordance with the teachings herein. Accordingly, embodiments of the present disclosure are not limited to that precisely as shown and described.

## Claims

1. A camera calibration method for X-ray imaging implemented on a computing device having one or more processors and one or more storage devices, the method comprising:
acquiring at least one image taken by a camera to be calibrated (160), wherein the at least one image includes a calibration target (510, 700, 810, 900), and the calibration target (510, 700, 810, 900) includes at least one calibration point;
selecting any calibration point of the at least one calibration point as a target calibration point (512);
determining, based on the at least one image, image coordinates of the target calibration point (512);
obtaining a first position and a second position of an X-ray imaging device (110, 400), wherein when the X-ray imaging device (110, 400) is located in the first position and the second position, the target calibration point (512) is within an imaging field of view of the X-ray imaging device (110, 400), and a first line and a second line are not parallel, the first line connecting a radiation source (430, 520) and a detector (440, 530) of the X-ray imaging device (110, 400) at the first position, the second line connecting the radiation source (430, 520) and the detector (440, 530) of the X-ray imaging device (110, 400) at the second position, when the X-ray imaging device (110, 400) is located at the first position and/or the second position, the first line and/or the second line overlaps with the target calibration point (512), and obtaining the first position of the X-ray imaging device (110, 400) includes:
acquiring a first image captured by the X-ray imaging device (110, 400) at a first candidate position, the first image including the target calibration point (512);
determining a first magnification based on the first image;
determining a first deviation between a first image position of the target calibration point (512) in the first image and an image center of the first image;
determining, based on the first magnification and the first deviation, a second deviation between a spatial position of the target calibration point (512) and the first line; and
determining the first position based on the second deviation;
determining, based on the first position and the second position, spatial coordinates of the target calibration point (512); and
performing calibration on the camera to be calibrated (160) based on the image coordinates and the spatial coordinates.

2. The method of claim1, wherein the first position is a position where the first line is perpendicular to a plane of the calibration target (510, 700, 810, 900), and the second position is a position where the second line is parallel to the plane of the calibration target (510, 700, 810, 900).

3. The method of claim 2, wherein determining, based on the first position and the second position, the spatial coordinates of the target calibration point (512) includes:
determining, based on the first position, first two-dimensional (2D) coordinates of the target calibration point (512) within the plane of the calibration target (510, 700, 810, 900);
determining, based on the second position, second 2D coordinates of the target calibration point (512) within a plane perpendicular to the plane of the calibration target (510, 700, 810, 900); and
determining, based on the first 2D coordinates and the second 2D coordinates, the spatial coordinates.

4. The method of claim 1, wherein
the first position corresponds to a first angle, and the second position corresponds to a second angle; and
determining, based on the first position and the second position, the spatial coordinates of the target calibration point (512) includes:
determining first coordinates of the first line based on the first position;
determining second coordinates of the second line based on the second position; and
determining the spatial coordinates based on the first coordinates and the second coordinates.

5. The method of any one of claims 1-4, wherein determining the first magnification based on the first image includes:
obtaining a size of at least part of the calibration target (510, 700, 810, 900) in the first image;
obtaining an actual size of the at least part of the calibration target (510, 700, 810, 900); and
determining the first magnification based on the size of the at least part of the calibration target (510, 700, 810, 900) in the first image and the actual size of the at least part of the calibration target (510, 700, 810, 900).

6. The method of any one of claims 1-4, wherein determining the first magnification based on the first image includes:
acquiring a third image captured by the X-ray imaging device (110, 400) at an auxiliary position, the third image including the target calibration point (512);
obtaining first coordinates related to the X-ray imaging device (110, 400) at the first candidate position;
obtaining second coordinates related to related to the X-ray imaging device (110, 400) at the auxiliary position;
obtaining a third coordinates of the target calibration point (512) in the first image;
obtaining a fourth coordinates of the target calibration point (512) in the third image; and
determining the first magnification based on the first coordinates, the second coordinates, the third coordinates, and the fourth coordinates.

7. The method of claim 6, wherein a line connecting the radiation source (430, 520) and the detector (440, 530) at the first candidate position and a line connecting the radiation source (430, 520) and the detector (440, 530) at the auxiliary position are parallel.

8. The method of claim 7, wherein the line connecting the radiation source (430, 520) and the detector (440, 530) at the first candidate position and the line connecting the radiation source (430, 520) and the detector (440, 530) at the auxiliary position are at an angle with a line vertical to a plane of the calibration target (510, 700, 810, 900).

9. The method of claim 8, wherein the first magnification is determined based further on the angle.

10. The method of any one of claims 1-9, wherein the obtaining the second position of the X-ray imaging device (110, 400) includes:
acquiring a second image captured by the X-ray imaging device (110, 400) at a second candidate position, the second image including the target calibration point (512);
determining a second magnification based on the second image;
determining a third deviation between a second image position of the target calibration point (512) in the second image and an image center of the second image;
determining, based on the second magnification and the third deviation, a fourth deviation between the spatial position of the target calibration point (512) and the second line;
determining the second position based on the fourth deviation.

11. The method of any one of claims 1-10, further includes:
acquiring a target image taken by the calibrated camera, wherein the image includes an object;
determining image coordinates of the object in the target image;
determining spatial coordinates of the object based on the image coordinates of the object and a calibration result of the calibrated camera; and
moving, based on the spatial coordinates of the object, the X-ray imaging device (110, 400) to a third position at which a center point of a third line connecting the radiation source (430, 520) and the detector (440, 530) of the X-ray imaging device (110, 400) overlaps the object.

12. The method of any one of claims 1-11, wherein the calibration target (510, 700, 810, 900) includes at least two calibration points, and the at least two calibration points are in spaced arrangement on an upper surface of a scanning bed of the X-ray imaging device (110, 400) to form at least one part of the calibration target (510, 700, 810, 900).

13. The method of any one of claims 1-12, wherein the calibration target (510, 700, 810, 900) includes at least one calibration unit (600, 711, 712, 721, 910, 920, 930, 940, 950, 960), each of the at least one calibration unit (600, 711, 712, 721, 910, 920, 930, 940, 950, 960) includes a calibration part (620) and a base plate (610), a geometric center (630) of the calibration part (620) is set as the calibration point, the calibration part (620) is disposed on the base plate (610), and there is an X-ray attenuation difference and/or an optical imaging difference between the calibration part (620) and the base plate (610).

14. A system, comprising:
at least one storage medium including a set of instructions;
at least one processor in communication with the at least one storage medium, wherein when executing the set of instructions, the at least one processor is directed to cause the system to perform operations including:
acquiring at least one image taken by a camera to be calibrated, wherein the at least one image includes a calibration target (510, 700, 810, 900), and the calibration target (510, 700, 810, 900) includes at least one calibration point;
selecting any calibration point of the at least one calibration point as a target calibration point (512);
determining, based on the at least one image, image coordinates of the target calibration point (512);
obtaining a first position and a second position of an X-ray imaging device (110, 400), wherein when the X-ray imaging device (110, 400) is located in the first position and the second position, the target calibration point (512) is within an imaging field of view of the X-ray imaging device (110, 400), and a first line and a second line are not parallel, the first line connecting a radiation source (430, 520) and a detector (440, 530) of the X-ray imaging device (110, 400) at the first position, the second line connecting the radiation source (430, 520) and the detector (440, 530) of the X-ray imaging device (110, 400) at the second position, when the X-ray imaging device (110, 400) is located at the first position and/or the second position, the first line and/or the second line overlaps with the target calibration point (512), and obtaining the first position of the X-ray imaging device (110, 400) includes:
acquiring a first image captured by the X-ray imaging device (110, 400) at a first candidate position, the first image including the target calibration point (512);
determining a first magnification based on the first image;
determining a first deviation between a first image position of the target calibration point (512) in the first image and an image center of the first image;
determining, based on the first magnification and the first deviation, a second deviation between a spatial position of the target calibration point (512) and the first line; and
determining the first position based on the second deviation;
determining, based on the first position and the second position, spatial coordinates of the target calibration point (512); and
performing calibration on the camera to be calibrated (160) based on the image coordinates and the spatial coordinates.

15. A non-transitory computer readable medium, comprising a set of instructions for data clustering, wherein when executed by at least one processor, the set of instructions direct the at least one processor to effectuate a method, the method comprising:
acquiring at least one image taken by a camera to be calibrated, wherein the at least one image includes a calibration target (510, 700, 810, 900), and the calibration target (510, 700, 810, 900) includes at least one calibration point;
selecting any calibration point of the at least one calibration point as a target calibration point (512);
determining, based on the at least one image, image coordinates of the target calibration point (512);
obtaining a first position and a second position of an X-ray imaging device (110, 400), wherein when the X-ray imaging device (110, 400) is located in the first position and the second position, the target calibration point (512) is within an imaging field of view of the X-ray imaging device (110, 400), and a first line and a second line are not parallel, the first line connecting a radiation source (430, 520) and a detector (440, 530) of the X-ray imaging device (110, 400) at the first position, the second line connecting the radiation source (430, 520) and the detector (440, 530) of the X-ray imaging device (110, 400) at the second position, when the X-ray imaging device (110, 400) is located at the first position and/or the second position, the first line and/or the second line overlaps with the target calibration point (512), and obtaining the first position of the X-ray imaging device (110, 400) includes:
acquiring a first image captured by the X-ray imaging device (110, 400) at a first candidate position, the first image including the target calibration point (512);
determining a first magnification based on the first image;
determining a first deviation between a first image position of the target calibration point (512) in the first image and an image center of the first image;
determining, based on the first magnification and the first deviation, a second deviation between a spatial position of the target calibration point (512) and the first line; and
determining the first position based on the second deviation;
determining, based on the first position and the second position, spatial coordinates of the target calibration point (512); and
performing calibration on the camera to be calibrated (160) based on the image coordinates and the spatial coordinates.

## Patentansprüche

1. Kamerakalibrierungsverfahren für die Röntgen-Bildgebung, das auf einer Computervorrichtung implementiert ist, die einen oder mehrere Prozessoren und eine oder mehrere Speichervorrichtungen aufweist, wobei das Verfahren umfasst:
Erfassen mindestens eines durch eine zu kalibrierende Kamera (160) aufgenommenen Bildes, wobei das mindestens eine Bild ein Kalibrierungsziel (510, 700, 810, 900) einschließt und das Kalibrierungsziel (510, 700, 810, 900) mindestens einen Kalibrierungspunkt einschließt;
Auswählen eines beliebigen Kalibrierungspunkts aus dem mindestens einen Kalibrierungspunkt als einen Ziel-Kalibrierungspunkt (512);
Bestimmen von Bildkoordinaten des Ziel-Kalibrierungspunkts (512) auf der Grundlage des mindestens einen Bildes;
Erhalten einer ersten Position und einer zweiten Position einer Röntgen-Bildgebungsvorrichtung (110, 400), wobei, wenn sich die Röntgen-Bildgebungsvorrichtung (110, 400) an der ersten Position und der zweiten Position befindet, der Ziel-Kalibrierungspunkt (512) innerhalb eines Bildaufnahmebereichs der Röntgenbildgebungsvorrichtung (110, 400) liegt und eine erste Linie und eine zweite Linie nicht parallel sind, wobei die erste Linie eine Strahlungsquelle (430, 520) und einen Detektor (440, 530) der Röntgen-Bildgebungsvorrichtung (110, 400) an der ersten Position verbindet und die zweite Linie die Strahlungsquelle (430, 520) und den Detektor (440, 530) der Röntgen-Bildgebungsvorrichtung (110, 400) an der zweiten Position verbindet, wobei, wenn sich die Röntgen-Bildgebungsvorrichtung (110, 400) an der ersten Position und/oder der zweiten Position befindet, die erste Linie und/oder die zweite Linie sich mit dem Ziel-Kalibrierungspunkt (512) überlappt, und wobei Erhalten der ersten Position der Röntgen-Bildgebungsvorrichtung (110, 400) einschließt:
Erfassen eines ersten Bildes, das durch die Röntgen-Bildgebungsvorrichtung (110, 400) an einer ersten Kandidatenposition aufgenommen wird, wobei das erste Bild den Ziel-Kalibrierungspunkt (512) einschließt;
Bestimmen einer ersten Vergrößerung auf der Grundlage des ersten Bildes;
Bestimmen einer ersten Abweichung zwischen einer ersten Bildposition des Ziel-Kalibrierungspunkts (512) im ersten Bild und einem Bildmittelpunkt des ersten Bildes;
Bestimmen einer zweiten Abweichung zwischen einer räumlichen Position des Ziel-Kalibrierungspunkts (512) und der ersten Linie auf der Grundlage der ersten Vergrößerung und der ersten Abweichung; und
Bestimmen der ersten Position auf der Grundlage der zweiten Abweichung;
Bestimmen von räumlichen Koordinaten des Ziel-Kalibrierungspunkts (512) auf der Grundlage der ersten Position und der zweiten Position; und
Durchführen der Kalibrierung an der zu kalibrierenden Kamera (160) auf der Grundlage der Bildkoordinaten und der räumlichen Koordinaten.

2. Verfahren nach Anspruch 1, wobei die erste Position eine Position ist, an der die erste Linie senkrecht zu einer Ebene des Kalibrierungsziels (510, 700, 810, 900) ist, und die zweite Position eine Position ist, an der die zweite Linie parallel zur Ebene des Kalibrierungsziels (510, 700, 810, 900) ist.

3. Verfahren nach Anspruch 2, wobei Bestimmen der räumlichen Koordinaten des Ziel-Kalibrierungspunkts (512) auf der Grundlage der ersten Position und der zweiten Position einschließt:
Bestimmen erster zweidimensionaler (2D-)Koordinaten des Ziel-Kalibrierungspunkts (512) innerhalb der Ebene des Kalibrierungsziels (510, 700, 810, 900) auf der Grundlage der ersten Position;
Bestimmen zweiter 2D-Koordinaten des Ziel-Kalibrierungspunkts (512) innerhalb einer Ebene senkrecht zur Ebene des Kalibrierungsziels (510, 700, 810, 900) auf der Grundlage der zweiten Position; und
Bestimmen der räumlichen Koordinaten auf der Grundlage der ersten 2D-Koordinaten und der zweiten 2D-Koordinaten.

4. Verfahren nach Anspruch 1, wobei:
die erste Position einem ersten Winkel entspricht und die zweite Position einem zweiten Winkel entspricht; und
Bestimmen der räumlichen Koordinaten des Ziel-Kalibrierungspunkts (512) auf der Grundlage der ersten Position und der zweiten Position einschließt:
Bestimmen erster Koordinaten der ersten Linie auf der Grundlage der ersten Position;
Bestimmen zweiter Koordinaten der zweiten Linie auf der Grundlage der zweiten Position; und
Bestimmen der räumlichen Koordinaten auf der Grundlage der ersten Koordinaten und der zweiten Koordinaten.

5. Verfahren nach einem der Ansprüche 1-4, wobei Bestimmen der ersten Vergrößerung auf der Grundlage des ersten Bildes einschließt:
Erhalten einer Größe mindestens eines Teils des Kalibrierungsziels (510, 700, 810, 900) im ersten Bild;
Erhalten einer tatsächlichen Größe des mindestens Teils des Kalibrierungsziels (510, 700, 810, 900); und
Bestimmen der ersten Vergrößerung auf der Grundlage der Größe des mindestens Teils des Kalibrierungsziels (510, 700, 810, 900) im ersten Bild und der tatsächlichen Größe des mindestens Teils des Kalibrierungsziels (510, 700, 810, 900).

6. Verfahren nach einem der Ansprüche 1-4, wobei Bestimmen der ersten Vergrößerung auf der Grundlage des ersten Bildes einschließt:
Erfassen eines dritten Bildes, das durch die Röntgen-Bildgebungsvorrichtung (110, 400) an einer Hilfsposition aufgenommen wird, wobei das dritte Bild den Ziel-Kalibrierungspunkt (512) einschließt;
Erhalten erster Koordinaten, die sich auf die Röntgen-Bildgebungsvorrichtung (110, 400) beziehen, an der ersten Kandidatenposition;
Erhalten zweiter Koordinaten, die sich auf die Röntgen-Bildgebungsvorrichtung (110, 400) beziehen, an der Hilfsposition;
Erhalten dritter Koordinaten des Ziel-Kalibrierungspunkts (512) im ersten Bild;
Erhalten vierter Koordinaten des Ziel-Kalibrierungspunkts (512) im dritten Bild; und
Bestimmen der ersten Vergrößerung auf der Grundlage der ersten Koordinaten, der zweiten Koordinaten, der dritten Koordinaten und der vierten Koordinaten.

7. Verfahren nach Anspruch 6, wobei eine Linie, welche die Strahlungsquelle (430, 520) und den Detektor (440, 530) an der ersten Kandidatenposition verbindet, und eine Linie, welche die Strahlungsquelle (430, 520) und den Detektor (440, 530) an der Hilfsposition verbindet, parallel sind.

8. Verfahren nach Anspruch 7, wobei die Linie, welche die Strahlungsquelle (430, 520) und den Detektor (440, 530) an der ersten Kandidatenposition verbindet, und die Linie, welche die Strahlungsquelle (430, 520) und den Detektor (440, 530) an der Hilfsposition verbindet, einen Winkel mit einer Linie bilden, die senkrecht zu einer Ebene des Kalibrierungsziels (510, 700, 810, 900) ist.

9. Verfahren nach Anspruch 8, wobei die erste Vergrößerung ferner auf der Grundlage des Winkels bestimmt wird.

10. Verfahren nach einem der Ansprüche 1-9, wobei das Erhalten der zweiten Position der Röntgen-Bildgebungsvorrichtung (110, 400) einschließt:
Erfassen eines zweiten Bildes, das durch die Röntgen-Bildgebungsvorrichtung (110, 400) an einer zweiten Kandidatenposition aufgenommen wird, wobei das zweite Bild den Ziel-Kalibrierungspunkt (512) einschließt;
Bestimmen einer zweiten Vergrößerung auf der Grundlage des zweiten Bildes;
Bestimmen einer dritten Abweichung zwischen einer zweiten Bildposition des Ziel-Kalibrierungspunkts (512) im zweiten Bild und einem Bildmittelpunkt des zweiten Bildes;
Bestimmen einer vierten Abweichung zwischen der räumlichen Position des Ziel-Kalibrierungspunkts (512) und der zweiten Linie auf der Grundlage der zweiten Vergrößerung und der dritten Abweichung;
Bestimmen der zweiten Position auf der Grundlage der vierten Abweichung.

11. Verfahren nach einem der Ansprüche 1-10, das ferner einschließt:
Erfassen eines durch die kalibrierten Kamera aufgenommenen Zielbildes, wobei das Bild ein Objekt einschließt;
Bestimmen von Bildkoordinaten des Objekts im Zielbild;
Bestimmen von räumlichen Koordinaten des Objekts auf der Grundlage der Bildkoordinaten des Objekts und eines Kalibrierungsergebnisses der kalibrierten Kamera; und
Bewegen der Röntgen-Bildgebungsvorrichtung (110, 400) auf der Grundlage der räumlichen Koordinaten des Objekts zu einer dritten Position, an der ein Mittelpunkt einer dritten Linie, welche die Strahlungsquelle (430, 520) und den Detektor (440, 530) der Röntgen-Bildgebungsvorrichtung (110, 400) verbindet, sich mit dem Objekt überlappt.

12. Verfahren nach einem der Ansprüche 1-11, wobei das Kalibrierungsziel (510, 700, 810, 900) mindestens zwei Kalibrierungspunkte einschließt und die mindestens zwei Kalibrierungspunkte in einem Abstand zueinander auf einer Oberseite eines Scan-Tischs der Röntgen-Bildgebungsvorrichtung (110, 400) angeordnet sind, um mindestens einen Teil des Kalibrierungsziels (510, 700, 810, 900) zu bilden.

13. Verfahren nach einem der Ansprüche 1-12, wobei das Kalibrierungsziel (510, 700, 810, 900) mindestens eine Kalibrierungseinheit (600, 711, 712, 721, 910, 920, 930, 940, 950, 960) einschließt, wobei jede der mindestens einen Kalibrierungseinheit (600, 711, 712, 721, 910, 920, 930, 940, 950, 960) einen Kalibrierungsteil (620) und eine Grundplatte (610) einschließt, wobei ein geometrischer Mittelpunkt (630) des Kalibrierungsteils (620) als der Kalibrierungspunkt festgelegt ist, der Kalibrierungsteil (620) auf der Grundplatte (610) angeordnet ist und zwischen dem Kalibrierungsteil (620) und der Grundplatte (610) ein Unterschied in der Röntgenstrahlungsabschwächung und/oder ein Unterschied bei der optischen Abbildung besteht.

14. System, umfassend:
mindestens ein Speichermedium, das eine Menge von Anweisungen einschließt;
mindestens einen Prozessor in Kommunikation mit dem mindestens einen Speichermedium, wobei der mindestens eine Prozessor bei der Ausführung der Menge von Anweisungen angewiesen wird, das System dazu zu veranlassen, Operationen durchzuführen, welche einschließen:
Erfassen mindestens eines durch eine zu kalibrierende Kamera aufgenommenen Bildes, wobei das mindestens eine Bild ein Kalibrierungsziel (510, 700, 810, 900) einschließt und das Kalibrierungsziel (510, 700, 810, 900) mindestens einen Kalibrierungspunkt einschließt;
Auswählen eines beliebigen Kalibrierungspunkts aus dem mindestens einen Kalibrierungspunkt als einen Ziel-Kalibrierungspunkt (512);
Bestimmen von Bildkoordinaten des Ziel-Kalibrierungspunkts (512) auf der Grundlage des mindestens einen Bildes;
Erhalten einer ersten Position und einer zweiten Position einer Röntgen-Bildgebungsvorrichtung (110, 400), wobei, wenn sich die Röntgen-Bildgebungsvorrichtung (110, 400) an der ersten Position und der zweiten Position befindet, der Ziel-Kalibrierungspunkt (512) innerhalb eines Abbildungssichtfeldes der Röntgenbildgebungsvorrichtung (110, 400) liegt und eine erste Linie und eine zweite Linie nicht parallel sind, wobei die erste Linie eine Strahlungsquelle (430, 520) und einen Detektor (440, 530) der Röntgen-Bildgebungsvorrichtung (110, 400) an der ersten Position verbindet und die zweite Linie die Strahlungsquelle (430, 520) und den Detektor (440, 530) der Röntgen-Bildgebungsvorrichtung (110, 400) an der zweiten Position verbindet, wobei, wenn sich die Röntgen-Bildgebungsvorrichtung (110, 400) an der ersten Position und/oder der zweiten Position befindet, die erste Linie und/oder die zweite Linie sich mit dem Ziel-Kalibrierungspunkt (512) überlappt, und wobei Erhalten der ersten Position der Röntgen-Bildgebungsvorrichtung (110, 400) einschließt:
Erfassen eines ersten Bildes, das durch die Röntgen-Bildgebungsvorrichtung (110, 400) an einer ersten Kandidatenposition aufgenommen wird, wobei das erste Bild den Ziel-Kalibrierungspunkt (512) einschließt;
Bestimmen einer ersten Vergrößerung auf der Grundlage des ersten Bildes;
Bestimmen einer ersten Abweichung zwischen einer ersten Bildposition des Ziel-Kalibrierungspunkts (512) im ersten Bild und einem Bildmittelpunkt des ersten Bildes;
Bestimmen einer zweiten Abweichung zwischen einer räumlichen Position des Ziel-Kalibrierungspunkts (512) und der ersten Linie auf der Grundlage der ersten Vergrößerung und der ersten Abweichung; und
Bestimmen der ersten Position auf der Grundlage der zweiten Abweichung;
Bestimmen von räumlichen Koordinaten des Ziel-Kalibrierungspunkts (512) auf der Grundlage der ersten Position und der zweiten Position; und
Durchführen der Kalibrierung an der zu kalibrierenden Kamera (160) auf der Grundlage der Bildkoordinaten und der räumlichen Koordinaten.

15. Nichtflüchtiges, computerlesbares Medium, eine Menge von Anweisungen für das Daten-Clustering umfassend, wobei die Menge von Anweisungen bei Ausführung durch mindestens einen Prozessor den mindestens einen Prozessor anweist, ein Verfahren zu durchzuführen, wobei das Verfahren umfasst:
Erfassen mindestens eines durch eine zu kalibrierende Kamera aufgenommenen Bildes, wobei das mindestens eine Bild ein Kalibrierungsziel (510, 700, 810, 900) einschließt und das Kalibrierungsziel (510, 700, 810, 900) mindestens einen Kalibrierungspunkt einschließt;
Auswählen eines beliebigen Kalibrierungspunkts aus dem mindestens einen Kalibrierungspunkt als einen Ziel-Kalibrierungspunkt (512);
Bestimmen von Bildkoordinaten des Ziel-Kalibrierungspunkts (512) auf der Grundlage des mindestens einen Bildes;
Erhalten einer ersten Position und einer zweiten Position einer Röntgen-Bildgebungsvorrichtung (110, 400), wobei, wenn sich die Röntgen-Bildgebungsvorrichtung (110, 400) an der ersten Position und der zweiten Position befindet, der Ziel-Kalibrierungspunkt (512) innerhalb eines Abbildungssichtfeldes der Röntgenbildgebungsvorrichtung (110, 400) liegt und eine erste Linie und eine zweite Linie nicht parallel sind, wobei die erste Linie eine Strahlungsquelle (430, 520) und einen Detektor (440, 530) der Röntgen-Bildgebungsvorrichtung (110, 400) an der ersten Position verbindet und die zweite Linie die Strahlungsquelle (430, 520) und den Detektor (440, 530) der Röntgen-Bildgebungsvorrichtung (110, 400) an der zweiten Position verbindet, wobei, wenn sich die Röntgen-Bildgebungsvorrichtung (110, 400) an der ersten Position und/oder der zweiten Position befindet, die erste Linie und/oder die zweite Linie sich mit dem Ziel-Kalibrierungspunkt (512) überlappt, und wobei Erhalten der ersten Position der Röntgen-Bildgebungsvorrichtung (110, 400) einschließt:
Erfassen eines ersten Bildes, das durch die Röntgen-Bildgebungsvorrichtung (110, 400) an einer ersten Kandidatenposition aufgenommen wird, wobei das erste Bild den Ziel-Kalibrierungspunkt (512) einschließt;
Bestimmen einer ersten Vergrößerung auf der Grundlage des ersten Bildes;
Bestimmen einer ersten Abweichung zwischen einer ersten Bildposition des Ziel-Kalibrierungspunkts (512) im ersten Bild und einem Bildmittelpunkt des ersten Bildes;
Bestimmen einer zweiten Abweichung zwischen einer räumlichen Position des Ziel-Kalibrierungspunkts (512) und der ersten Linie auf der Grundlage der ersten Vergrößerung und der ersten Abweichung; und
Bestimmen der ersten Position auf der Grundlage der zweiten Abweichung;
Bestimmen von räumlichen Koordinaten des Ziel-Kalibrierungspunkts (512) auf der Grundlage der ersten Position und der zweiten Position; und
Durchführen der Kalibrierung an der zu kalibrierenden Kamera (160) auf der Grundlage der Bildkoordinaten und der räumlichen Koordinaten.

## Revendications

1. Procédé d'étalonnage de caméra pour une imagerie par rayons X, mis en œuvre sur un dispositif informatique présentant un ou plusieurs processeurs et un ou plusieurs dispositifs de stockage, le procédé comprenant :
l'acquisition d'au moins une image prise par une caméra à étalonner (160), dans lequel la au moins une image inclut une cible d'étalonnage (510, 700, 810, 900), et la cible d'étalonnage (510, 700, 810, 900) inclut au moins un point d'étalonnage ;
la sélection d'un quelconque point d'étalonnage parmi le au moins un point d'étalonnage comme point d'étalonnage cible (512) ;
la détermination, sur la base de la au moins une image, de coordonnées d'image du point d'étalonnage cible (512) ;
l'obtention d'une première position et d'une deuxième position d'un dispositif d'imagerie par rayons X (110, 400), dans lequel lorsque le dispositif d'imagerie par rayons X (110, 400) est situé dans la première position ou la deuxième position, le point d'étalonnage cible (512) se trouve dans un champ de vision d'imagerie du dispositif d'imagerie par rayons X (110, 400), et une première ligne et une deuxième ligne ne sont pas parallèles, la première ligne reliant une source de rayonnement (430, 520) et un détecteur (440, 530) du dispositif d'imagerie par rayon X (110, 400) dans la première position, la deuxième ligne reliant la source de rayonnement (430, 520) au détecteur (440, 530) du dispositif d'imagerie par rayons X (110, 400) dans la deuxième position, lorsque le dispositif d'imagerie par rayons X (110, 400) est situé dans la première position et/ou dans la deuxième position, la première ligne et/ou la deuxième ligne chevauche le point d'étalonnage cible (512), et l'obtention de la première position du dispositif d'imagerie par rayons X (110, 400) inclut :
l'acquisition d'une première image capturée par le dispositif d'imagerie par rayons X (110, 400) dans une première position candidate, la première image incluant le point d'étalonnage cible (512) ;
la détermination d'un premier grossissement sur la base de la première image ;
la détermination d'un premier écart entre une première position d'image du point d'étalonnage cible (512) dans la première image et un centre d'image de la première image ;
la détermination, sur la base du premier grossissement et du premier écart, d'un deuxième écart entre une position spatiale du point d'étalonnage cible (512) et la première ligne ; et
la détermination de la première position sur la base du deuxième écart ;
la détermination, sur la base de la première position et de la deuxième position, de coordonnées spatiales du point d'étalonnage cible (512) ; et
la réalisation de l'étalonnage de la caméra à étalonner (160) sur la base des coordonnées d'image et des coordonnées spatiales.

2. Procédé selon la revendication 1, dans lequel la première position est une position où la première ligne est perpendiculaire à un plan de la cible d'étalonnage (510, 700, 810, 900), et la deuxième position est une position où la deuxième ligne est parallèle au plan de la cible d'étalonnage (510, 700, 810, 900).

3. Procédé selon la revendication 2, dans lequel la détermination, sur la base de la première et de la deuxième position, des coordonnées spatiales du point d'étalonnage cible (512) inclut :
la détermination, sur la base de la première position, des premières coordonnées bidimensionnelles (2D) du point d'étalonnage cible (512) dans le plan de la cible d'étalonnage (510, 700, 810, 900) ;
la détermination, sur la base de la deuxième position, de deuxièmes coordonnées 2D du point d'étalonnage cible (512) dans un plan perpendiculaire au plan de la cible d'étalonnage (510, 700, 810, 900) ; et
la détermination, sur la base des premières coordonnées 2D et des deuxièmes coordonnées 2D, des coordonnées spatiales.

4. Procédé selon la revendication 1, dans lequel
la première position correspond à un premier angle, et la deuxième position correspond à un deuxième angle ; et
la détermination, sur la base de la première position et de la deuxième position, des coordonnées spatiales du point d'étalonnage cible (512) inclut :
la détermination de premières coordonnées de la première ligne sur la base de la première position ;
la détermination de deuxièmes coordonnées de la deuxième ligne sur la base de la deuxième position ; et
la détermination des coordonnées spatiales sur la base des premières coordonnées et des deuxièmes coordonnées.

5. Procédé selon l'une quelconque des revendications 1-4, dans lequel la détermination du premier grossissement sur la base de la première image inclut :
l'obtention d'une taille d'au moins une partie de la cible d'étalonnage (510, 700, 810, 900) dans la première image ;
l'obtention d'une taille réelle de la au moins une partie de la cible d'étalonnage (510, 700, 810, 900) ; et
la détermination du premier grossissement sur la base de la taille de la au moins une partie de la cible d'étalonnage (510, 700, 810, 900) dans la première image et de la taille réelle de la au moins une partie de la cible d'étalonnage (510, 700, 810, 900).

6. Procédé selon l'une quelconque des revendications 1-4, dans lequel la détermination du premier grossissement sur la base de la première image inclut :
l'acquisition d'une troisième image capturée par le dispositif d'imagerie par rayons X (110, 400) dans une position auxiliaire, la troisième image incluant le point d'étalonnage cible (512) ;
l'obtention de premières coordonnées relatives au dispositif d'imagerie par rayons X (110, 400) dans la première position candidate ;
l'obtention de deuxièmes coordonnées liées au dispositif d'imagerie par rayons X (110, 400) dans la position auxiliaire ;
l'obtention de troisièmes coordonnées du point d'étalonnage cible (512) dans la première image ;
l'obtention de quatrièmes coordonnées du point d'étalonnage cible (512) dans la troisième image ; et
la détermination du premier grossissement sur la base des premières coordonnées, des deuxièmes coordonnées, des troisièmes coordonnées et des quatrièmes coordonnées.

7. Procédé selon la revendication 6, dans lequel une ligne reliant la source de rayonnement (430, 520) et le détecteur (440, 530) dans la première position candidate et une ligne reliant la source de rayonnement (430, 520) et le détecteur (440, 530) dans la position auxiliaire sont parallèles.

8. Procédé selon la revendication 7, dans lequel la ligne reliant la source de rayonnement (430, 520) et le détecteur (440, 530) dans la première position candidate et la ligne reliant la source de rayonnement (430, 520) et le détecteur (440, 530) dans la position auxiliaire sont à un angle avec une ligne verticale à un plan de la cible d'étalonnage (510, 700, 810, 900).

9. Procédé selon la revendication 8, dans lequel le premier grossissement est déterminé en outre sur la base de l'angle.

10. Procédé selon l'une quelconque des revendications 1-9, dans lequel l'obtention de la deuxième position du dispositif d'imagerie par rayons X (110, 400) inclut :
l'acquisition d'une deuxième image capturée par le dispositif d'imagerie par rayons X (110, 400) dans une deuxième position candidate, la deuxième image incluant le point d'étalonnage cible (512) ;
la détermination d'un deuxième grossissement sur la base de la deuxième image ;
la détermination d'un troisième écart entre une deuxième position d'image du point d'étalonnage cible (512) dans la deuxième image et un centre d'image de la deuxième image ;
la détermination, sur la base du deuxième grossissement et du troisième écart, d'un quatrième écart entre la position spatiale du point d'étalonnage cible (512) et la deuxième ligne ;
la détermination de la deuxième position sur la base du quatrième écart.

11. Procédé selon l'une quelconque des revendications 1-10, incluant en outre :
l'acquisition d'une image cible prise par la caméra étalonnée, dans lequel l'image inclut un objet ;
la détermination de coordonnées d'image de l'objet dans l'image cible ;
la détermination de coordonnées spatiales de l'objet sur la base des coordonnées d'image de l'objet et d'un résultat d'étalonnage de la caméra étalonnée ; et
le déplacement, sur la base des coordonnées spatiales de l'objet, le dispositif d'imagerie par rayons X (110, 400) vers une troisième position où un point central d'une troisième ligne reliant la source de rayonnement (430, 520) et le détecteur (440, 530) du dispositif d'imagerie par rayons X (110, 400) chevauche l'objet.

12. Procédé selon l'une quelconque des revendications 1 à 11, dans lequel la cible d'étalonnage (510, 700, 810, 900) inclut au moins deux points d'étalonnage, et les au moins deux points d'étalonnage sont disposés dans un agencement espacé sur une surface supérieure d'un lit de balayage du dispositif d'imagerie par rayons X (110, 400) pour former au moins une partie de la cible d'étalonnage (510, 700, 810, 900).

13. Procédé selon l'une quelconque des revendications 1-12, dans lequel la cible d'étalonnage (510, 700, 810, 900) inclut au moins une unité d'étalonnage (600, 711, 712, 721, 910, 920, 930, 940, 950, 960), chacune de la au moins une unité d'étalonnage (600, 711, 712, 721, 910, 920, 930, 940, 950, 960) inclut une partie d'étalonnage (620) et une plaque de base (610), un centre géométrique (630) de la partie d'étalonnage (620) est défini comme point d'étalonnage, la partie d'étalonnage (620) est disposée sur la plaque de base (610), et il existe une différence d'atténuation des rayons X et/ou une différence d'imagerie optique entre la partie d'étalonnage (620) et la plaque de base (610).

14. Système, comprenant :
au moins un support de stockage incluant un ensemble d'instructions ;
au moins un processeur en communication avec le au moins un support de stockage, dans lequel, lors de l'exécution de l'ensemble d'instructions, le au moins un processeur est dirigé pour amener le système à effectuer des opérations incluant :
l'acquisition d'au moins une image prise par une caméra à étalonner, dans lequel la au moins une image inclut une cible d'étalonnage (510, 700, 810, 900), et la cible d'étalonnage (510, 700, 810, 900) inclut au moins un point d'étalonnage ;
la sélection d'un quelconque point d'étalonnage parmi le au moins un point d'étalonnage comme point d'étalonnage cible (512) ;
la détermination, sur la base de la au moins une image, de coordonnées d'image du point d'étalonnage cible (512) ;
l'obtention d'une première position et d'une deuxième position d'un dispositif d'imagerie par rayons X (110, 400), dans lequel lorsque le dispositif d'imagerie par rayons X (110, 400) est situé dans la première position et la deuxième position, le point d'étalonnage cible (512) se trouve dans un champ de vision d'imagerie du dispositif d'imagerie par rayons X (110, 400), et une première ligne et une deuxième ligne ne sont pas parallèles, la première ligne reliant une source de rayonnement (430, 520) et un détecteur (440, 530) du dispositif d'imagerie par rayon X (110, 400) dans la première position, la deuxième ligne reliant la source de rayonnement (430, 520) et le détecteur (440, 530) du dispositif d'imagerie par rayons X (110, 400) dans la deuxième position, lorsque le dispositif d'imagerie par rayons X (110, 400) est situé dans la première position et/ou dans la deuxième position, la première ligne et/ou la deuxième ligne chevauche le point d'étalonnage cible (512), et l'obtention de la première position du dispositif d'imagerie par rayons X (110, 400) inclut :
l'acquisition d'une première image capturée par le dispositif d'imagerie par rayons X (110, 400) dans une première position candidate, la première image incluant le point d'étalonnage cible (512) ;
la détermination d'un premier grossissement sur la base de la première image ;
la détermination d'un premier écart entre une première position d'image du point d'étalonnage cible (512) dans la première image et un centre d'image de la première image ;
la détermination, sur la base du premier grossissement et du premier écart, d'un deuxième écart entre une position spatiale du point d'étalonnage cible (512) et la première ligne ; et
la détermination de la première position sur la base du deuxième écart ;
la détermination, sur la base de la première position et de la deuxième position, de coordonnées spatiales du point d'étalonnage cible (512) ; et
la réalisation de l'étalonnage de la caméra à étalonner (160) sur la base des coordonnées d'image et des coordonnées spatiales.

15. Support non transitoire lisible par ordinateur, comprenant un ensemble d'instructions pour un regroupement de données, dans lequel, lorsqu'il est exécuté par au moins un processeur, l'ensemble d'instructions amène le au moins un processeur à effectuer un procédé, le procédé comprenant :
l'acquisition d'au moins une image prise par une caméra à étalonner, dans lequel la au moins une image inclut une cible d'étalonnage (510, 700, 810, 900), et la cible d'étalonnage (510, 700, 810, 900) inclut au moins un point d'étalonnage ;
la sélection d'un quelconque point d'étalonnage parmi le au moins un point d'étalonnage comme point d'étalonnage cible (512) ;
la détermination, sur la base de la au moins une image, de coordonnées d'image du point d'étalonnage cible (512) ;
l'obtention d'une première position et d'une deuxième position d'un dispositif d'imagerie par rayons X (110, 400), dans lequel lorsque le dispositif d'imagerie par rayons X (110, 400) est situé dans la première position et la deuxième position, le point d'étalonnage cible (512) se trouve dans un champ de vision d'imagerie du dispositif d'imagerie par rayons X (110, 400), et une première ligne et une deuxième ligne ne sont pas parallèles, la première ligne reliant une source de rayonnement (430, 520) et un détecteur (440, 530) du dispositif d'imagerie par rayon X (110, 400) dans la première position, la deuxième ligne reliant la source de rayonnement (430, 520) et le détecteur (440, 530) du dispositif d'imagerie par rayons X (110, 400) dans la deuxième position, lorsque le dispositif d'imagerie par rayons X (110, 400) est situé dans la première position et/ou dans la deuxième position, la première ligne et/ou la deuxième ligne chevauche le point d'étalonnage cible (512), et l'obtention de la première position du dispositif d'imagerie par rayons X (110, 400) inclut :
l'acquisition d'une première image capturée par le dispositif d'imagerie par rayons X (110, 400) dans une première position candidate, la première image incluant le point d'étalonnage cible (512) ;
la détermination d'un premier grossissement sur la base de la première image ;
la détermination d'un premier écart entre une première position d'image du point d'étalonnage cible (512) dans la première image et un centre d'image de la première image ;
la détermination, sur la base du premier grossissement et du premier écart, d'un deuxième écart entre une position spatiale du point d'étalonnage cible (512) et la première ligne ; et
la détermination de la première position sur la base du deuxième écart ;
la détermination, sur la base de la première position et de la deuxième position, de coordonnées spatiales du point d'étalonnage cible (512) ; et
la réalisation de l'étalonnage de la caméra à étalonner (160) sur la base des coordonnées d'image et des coordonnées spatiales.
